# EUROPEAN PATENT APPLICATION

(11) **EP 4 725 430 A2**
(43) Date of publication of application: **15.04.2026**
(21) Application number: 26161637.9
(22) Date of filing: 18.06.2018
(51) Int. Cl.: A61B 18/00

(54) **APPARATUSES FOR HIGH DENSITY SENSING AND ABLATION DURING A MEDICAL PROCEDURE**

(30) Priority: 19.06.2017 US 201762521983 P
(62) Divisional of application: 18739683.3
(71) Applicant: St. Jude Medical, Cardiology Division, Inc., St. Paul, MN 55117 (US)
(72) Inventor: OLSON, Gregory K., Elk River, Minnesota, 55330 (US)
(74) Representative: Mathys & Squire

(57) **Abstract**

A medical device comprising a first shaping element, a second shaping element located distally with respect to the first shaping element, a support structure that extends between the first and the second shaping elements, where each of the first and the second shaping element s are transversely oriented with respect to a longitudinal axis that extends through a center of each shaping element, and a plurality of interactive elements. An apparatus for an elongate medical device comprising a flexible planar substrate, a support structure, and a plurality of interactive elements. A helical medical device comprising a first planar substrate, wherein the first planar substrate has a helical shape and a second planar substrate coupled with the first planar substrate, wherein the second planar substrate includes a plurality of interactive elements.

## Description

### CROSS-REFERENCE TO RELATED APPLICATIONS

This application claims the benefit of United States provisional application no. 62/521,983, filed 19 June 2017, which is hereby incorporated by reference as though fully set forth herein.

### BACKGROUND

### a. Field

Embodiments of the present disclosure relate to apparatuses and methods for monitoring contact with cardiac tissue and cardiac electrical activity, and for ablating tissue. In particular, embodiments of the present disclosure relate to elongate medical devices comprising one or more substrates that are expandable from a first shape to a second shape, and to a plurality of interactive elements that are located on the substrate, and to methods of using such devices and elements.

### b. Background Art

Electrophysiology catheters are used in a variety of diagnostic and/or therapeutic medical procedures to address conditions such as atrial arrhythmia, including for example, ectopic atrial tachycardia, atrial fibrillation, and atrial flutter. Arrhythmia can create a variety of dangerous conditions including irregular heart rates, loss of synchronous atrioventricular contractions and stasis of blood flow which can lead to a variety of ailments and even death.

In a typical procedure, a catheter is manipulated through a patient's vasculature to, for example, a patient's heart, and carries one or more electrodes which may be used for diagnosis, mapping, ablation, or other treatments. Once at the intended site, treatment may involve radio frequency (RF) ablation, cryoablation, lasers, chemicals, high-intensity focused ultrasound, etc. An ablation catheter imparts such ablative energy to cardiac tissue to create a lesion in the cardiac tissue. This lesion disrupts undesirable electrical pathways and thereby limits or prevents stray electrical signals that lead to arrhythmias. As readily apparent, such treatment requires precise control of the catheter during delivery to and use at the treatment site, which can invariably be a function of a user's skill level.

Some prior practices effect multiple ablations on tissue to create a lesion line. For example, some prior practices for effecting multiple ablations on tissue involve performing a first ablation at a first point with an ablation catheter, then performing a second ablation at a second point with the ablation catheter, and then performing a third ablation at a third site with the ablation catheter, and so on. Thus, a number of single point ablations are made, often adjacent to one another, to create the lesion line. A frequent location for ablation lines is around/between the pulmonary veins in the left atrium of the heart. However, practices such as this can result in a user having to ensure that the ablation sites are adjacent to one another and also that sufficient contact is established at each ablation site.

### BRIEF SUMMARY

The instant disclosure, in at least one embodiment, relates to a flexible catheter structure comprising a first shaping element, a second shaping element located distal of the first shaping element, wherein each of the first and the second shaping elements is expandable from a contracted state to an expanded state and are transversely oriented, when in the expanded state, with respect to a longitudinal axis that extends through a center of each shaping element. The apparatus also comprises a support structure that extends between the first shaping element and the second shaping element and an interactive element.

In another embodiment, an apparatus for an elongate medical device comprises a flexible substrate, wherein the flexible substrate is coupled with a distal end of the elongate medical device and is configured to have an expanded state and a collapsed state, a support structure, wherein the support structure is coupled with the flexible substrate, and a plurality of interactive elements, wherein the plurality of interactive elements are arranged in a pattern on the flexible substrate.

In yet another embodiment, a helical medical device comprises a first planar substrate, where the first planar substrate has a helical shape, and an interactive element, wherein the interactive element is coupled with the first planar substrate, and a second planar substrate coupled with the first planar substrate, wherein the second planar substrate includes a plurality of interactive elements.

In another embodiment, an apparatus comprises a support structure located at the shaft distal end, wherein the support structure is expandable from a contracted state to an expanded state with respect to the longitudinal axis; a flexible planar substrate coupled with the support structure at an outer surface formed by the support structure, and a plurality of interactive elements wherein the plurality of interactive elements are coupled with the flexible planar substrate.

### BRIEF DESCRIPTION OF THE DRAWINGS

FIG. 1 is a schematic system diagram showing a medical device and a medical positioning system, in accordance with embodiments of the present disclosure.
FIG. 2 is a block diagram of a system for performing a medical procedure, in accordance with embodiments of the present disclosure.
FIG. 3A is a side view of a flexible catheter structure in an expanded state, in accordance with embodiments of the present disclosure.
FIG. 3B is a side view of the flexible catheter structure from Fig. 3A in a contracted state, in accordance with embodiments of the present disclosure.
FIG. 3C is a side view of a flexible catheter structure similar to FIG. 3A with additional annular structure, in accordance with embodiments of the present disclosure.
FIG. 4 is a side view of another embodiment of a flexible catheter structure, in accordance with embodiments of the present disclosure.
FIGS. 5A-5F are isometric side and distal end views of exemplary configurations of a flexible catheter structure in various states of expansion, in accordance with embodiments of the present disclosure.
FIG. 6 is an isometric side and proximal end view of an exemplary embodiment of a flexible catheter structure, in accordance with embodiments of the present disclosure.
FIG. 7 depicts an isometric side and distal end view a flexible catheter structure that can include a first shaping element and a second shaping element, shown juxtaposed prior to use during a medical procedure, in accordance with embodiments of the present disclosure.
FIG. 8 is an isometric side and distal end view of a flexible catheter structure that can include an alternative shaping element comprising, a plurality of shaping hoops that are connected in this embodiment, where the plurality of shaping hoops comprises a first or proximal shaping hoop and a second or distal shaping hoop, in accordance with embodiments of the present disclosure.
FIG. 9A is an isometric side and distal end view of a flexible catheter support structure that includes the first shaping element and the second shaping element, as shown in FIG. 7, being located in a pulmonary vein (PV), in accordance with embodiments of the present disclosure.
FIG. 9B is an isometric side and distal end view of a flexible catheter support structure that depicts a single elongate element that forms a first shaping hoop and a second shaping hoop, similar to FIG. 8, in a PV, in accordance with embodiments of the present disclosure.
FIG. 10 is an isometric side view of a flexible catheter structure comprising an anatomically configured tissue shaping element that includes a first shaping hoop and a second shaping hoop together with a support structure (flexible substrate) extending there between, , in accordance with embodiments of the present disclosure.
FIG. 11 is an isometric side and distal end view of a flexible catheter structure comprising an anatomically configured tissue shaping element used to form the shaping hoop and the second shaping hoop of FIG. 10, in accordance with embodiments of the present disclosure.
FIG. 12 is a proximal end view of the flexible catheter structure depicted in FIGS. 10 and 11, in accordance with embodiments of the present disclosure.
FIG. 13 is a cross-sectional front view of a portion of a heart with a flexible catheter structure comprising the anatomically configured tissue shaping element of FIGS. 10-12 about to be located in a pulmonary vein, in accordance with embodiments of the present disclosure
FIG. 14 is a cross-sectional front view of a heart with the with the flexible catheter structure comprising the anatomically configured tissue shaping element of FIGS. 10-12 positioned within the right superior pulmonary vein, prior to deployment of the tissue shaping element, in accordance with embodiments of the present disclosure
FIG. 15 is an enlarged cross-sectional front view of a pulmonary vein with the anatomically configured tissue shaping element of FIGS. 10-12 positioned therein, prior to deployment of the anatomically configured device, in accordance with embodiments of the present disclosure
FIG. 16 is a cross-sectional front view similar to that depicted in FIG. 15 but showing the anatomically configured tissue shaping element of FIGS. 10-12 deployed therein, in accordance with embodiments of the present disclosure
FIG. 17A is a side view of a flexible catheter structure comprising a transverse planar array of sensors, in accordance with embodiments of the present disclosure.
FIG. 17B is a cross-sectional side view of a graphical representation of a human heart with an embodiment of the transverse planar array of sensors depicted in FIG. 17A, in accordance with embodiments of the present disclosure.
FIG. 18A is an isometric view of a helical medical device comprising a first substrate and a plurality of interactive elements in an expanded state, in accordance with embodiments of the present disclosure.
FIG. 18B is an isometric view of the helical medical device of FIG. 18A in a contracted state, in accordance with embodiments of the present disclosure.
FIG. 18C is a cross-sectional view of the helical medical device of FIGS. 18A-B, in accordance with embodiments of the present disclosure.
FIG. 19A is an isometric view of a helical medical device comprising a first substrate, a second substrate, and a plurality of interactive elements in a contracted state, in accordance with embodiments of the present disclosure.
FIG. 19B is an isometric view of the helical medical device of FIG. 19A in a contracted state, in accordance with embodiments of the present disclosure.
FIG. 19C is a cross-sectional view of the helical medical device of FIGS. 19A- B, in accordance with embodiments of the present disclosure.

### DETAILED DESCRIPTION OF EMBODIMENTS

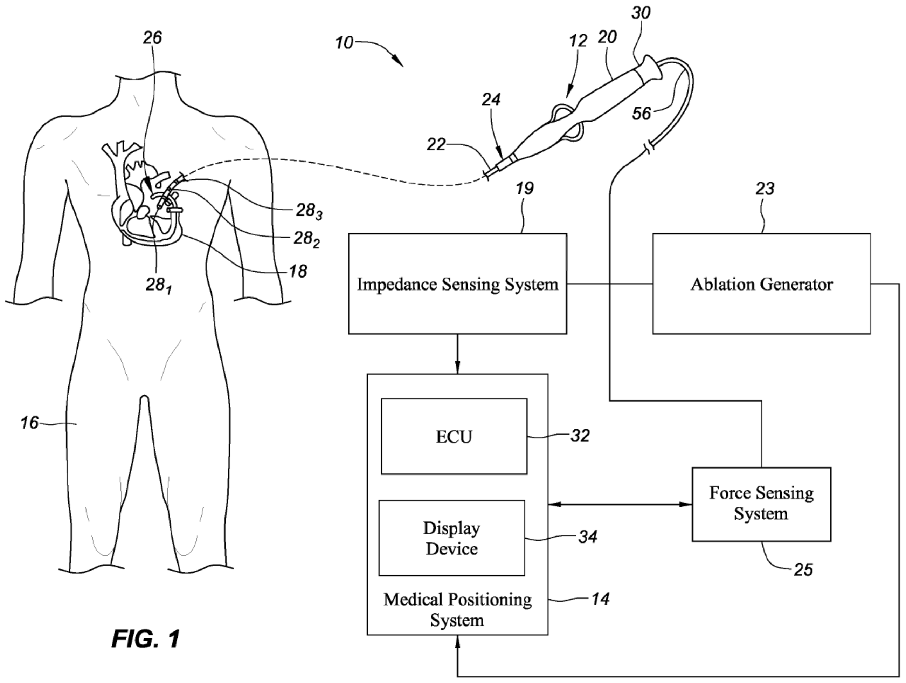

In some embodiments, and with reference to FIG. 1, the system 10 can include a medical device 12 and a medical positioning system 14. The medical device 12 can include an elongate medical device such as, for example and without limitation, a catheter, or a sheath, introducer, endoscope, or other device configured for insertion into the body. For purposes of illustration and clarity, the description below will be limited to an embodiment wherein the medical device 12 comprises a catheter (a sample catheter is shown in FIG. 1 (e.g., catheter 12). It will be appreciated, however, that the present disclosure is not meant to be limited to catheters and may also be or include a guidewire, an introducer, or some other elongate medical device.

With continued reference to FIG. 1, the catheter 12 can be configured to be inserted into a patient's body 16, and more particularly, into the patient's heart 18. The catheter 12 can include, for example, a handle 20 that has a proximal end 30, a shaft 22 having a proximal end portion 24 and a distal end portion 26, and one or more sensors 28 mounted in or on the shaft 22 of the catheter 12. As used herein, "sensor 28" or "sensors 28" may refer to one or more sensors 28₁, 28₂, ... 28_{N}, as appropriate and as generally depicted. In an exemplary embodiment, the sensors 28 are disposed at the distal end portion 26 of the shaft 22. The catheter 12 may further include other conventional components such as, for example and without limitation, a temperature sensor, additional sensors or electrodes, ablation elements (e.g., ablation tip electrodes for delivering RF ablative energy, high intensity focused ultrasound ablation elements, etc.), and corresponding conductors or leads.

The shaft 22 can be an elongate, tubular, flexible member configured for movement within the body 16. The shaft 22 supports, for example and without limitation, sensors and/or electrodes mounted thereon, such as, for example, the sensors 28, associated conductors, and possibly additional electronics used for signal processing and conditioning. The shaft 22 may also permit transport, delivery, and/or removal of fluids (including irrigation fluids, cryogenic ablation fluids, and bodily fluids), medicines, and/or surgical tools or instruments. The shaft 22 may be made from conventional materials such as polyurethane, and define one or more lumens configured to house and/or transport electrical conductors, fluids, or surgical tools. The shaft 22 may be introduced into a blood vessel or other structure within the body 16 through a conventional introducer. The shaft 22 may then be steered or guided through the body 16 to a desired location, such as the heart 18, using means well known in the art.

The sensors 28 mounted in or on the shaft 22 of the catheter 12 may be provided for a variety of diagnostic and therapeutic purposes including, for example and without limitation, electrophysiological studies, pacing, cardiac mapping, and ablation. In an exemplary embodiment, one or more of the sensors 28 are provided to perform a location or position sensing function. More particularly, and as will be described in greater detail below, one or more of the sensors 28 are configured to be a positioning sensor that provides information relating to the location (e.g., position and orientation) of the catheter 12, and the distal end portion 26 of the shaft 22 thereof, in particular, at certain points in time. Accordingly, in such an embodiment, as the catheter 12 is moved along a surface of a structure of interest of the heart 18 and/ or about the interior of the structure, the sensor(s) 28 can be used to collect location data points that correspond to the surface of, and/or other locations within, the structure of interest. These location data points can then be used for a number of purposes such as, for example and without limitation, the construction of surface models of the structure of interest.

For purposes of clarity and illustration, the description below will be with respect to an embodiment wherein a single sensor 28 of the catheter 12 comprises a positioning sensor. It will be appreciated, however, that in other exemplary embodiments, which remain within the spirit and scope of the present disclosure, the catheter 12 may comprise more than one positioning sensor as well as other sensors or electrodes configured to perform other diagnostic and/or therapeutic functions. As will be described in greater detail below, the sensor 28 can include a pair of leads extending from a sensing element thereof (e.g., a coil) that are configured to electrically couple the sensor 28 to other components of the system 10, such as, for example, the medical positioning system 14. In some embodiments, the sensing element can be an electromagnetic position sensor, such as a sensor coil, which can sense a magnetic field that is generated in proximity to the patient. Depending on a position and orientation (P&O) of the electromagnetic position sensor, different electrical signals can be generated by the coil and transferred to the medical positioning system, for a determination of a location reading that can be indicative of the P&O of the electromagnetic position sensor.

The location readings may each include at least one or both of a position and an orientation (P&O) relative to a reference coordinate system, which may be the coordinate system of medical positioning system 14. For some types of sensors, the P&O may be expressed with five degrees-of-freedom (five DOF) as a three-dimensional (3D) position (*i.e*., a coordinate in three axes X, Y and Z) and two-dimensional (2D) orientation (*e*.*g*., an azimuth and elevation) of sensor 28 in a magnetic field relative to a magnetic field generator(s) or transmitter(s) and/or a plurality of electrodes in an applied electrical field relative to an electrical field generator (e.g., a set of electrode patches). For other sensor types, the P&O may be expressed with six degrees-of-freedom (six DOF) as a 3D position (*i.e*., X, Y, Z coordinates) and 3D orientation (*i.e*., roll, pitch, and yaw).

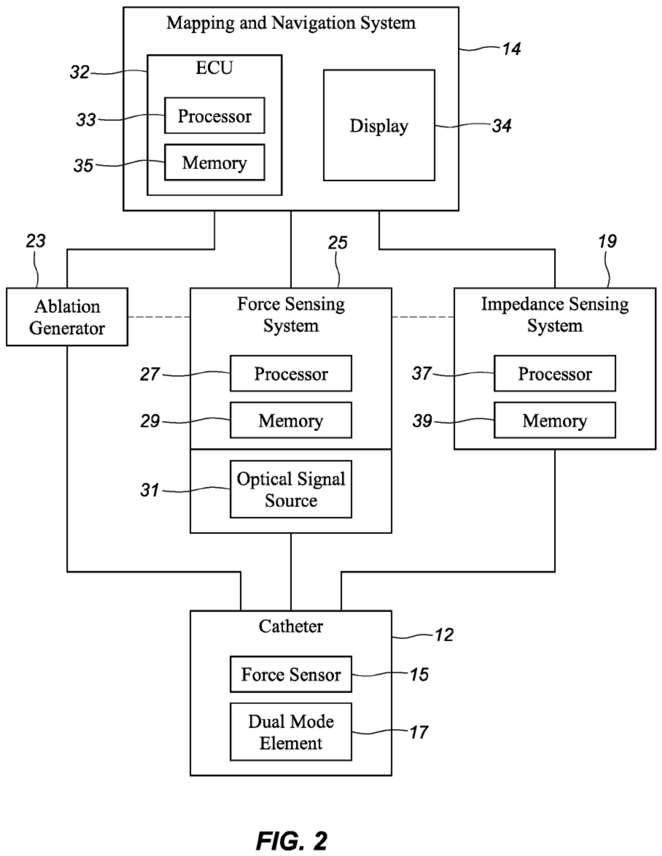

FIG. 2 is a block diagram view of an exemplary embodiment of a system 10 for performing a medical procedure. The system 10 may be used, for example, to perform an ablation procedure on tissue 18 of a patient, such as the heart of the patient. The system 10 may include a mapping and navigation system 14, an ablation generator 23, a force sensing system 25, an impedance sensing system 19, and a catheter 12.

The catheter 12 may include, in an embodiment, various components for performing an ablation procedure, including components for delivering ablation energy and making various measurements relevant to the delivery of the ablation energy. For example, the catheter 12 may include a force sensor 15 and a plurality of interactive elements 17, in an embodiment. In addition, the catheter 12 may include a handle 20, a shaft 22, and other components. Further description of the systems and components are contained in U.S. provisional patent application 62/331,398 filed on 03 May 2016, which is hereby incorporated by reference in its entirety as though fully set forth herein.

With continued reference to FIGS. 1 and 2, the plurality of interactive elements 17 (FIG. 2) may be provided for the catheter 12 to deliver ablation energy to tissue 18 of a body 16. In addition to the plurality of interactive elements 17 (FIG. 2), one or more additional electrodes can be included on the catheter 12 which may include a ring electrode, tip electrode, spot electrode, etc.

The impedance sensing system 19 (FIG. 2) may be provided for measuring tissue impedance. The impedance sensing system 19 may be, or may include, an electrode (e.g., the plurality of interactive elements 17 (FIG. 2), two or more patch electrodes) and an impedance sensor. In an embodiment in which the impedance sensing system 19 generates a signal, a current may be driven between, for example, one of the plurality of interactive elements 17 and a second electrode (e.g., a patch electrode), as further described below, to determine an impedance of the tissue 18, which may be a complex impedance. The impedance of the tissue 18 can be measured, for example, using a patch electrode at a location on the body 16 such as at the neck, a location of the chest or a location on the leg of the body. The impedance of the tissue 18 can also be measured using one or more electrodes (e.g., multi-electrode impedance or dipole) similar to the description in U.S. Patent Application Publication No. 2014/0364715, the entirety of which is incorporated herein by reference as though fully set for herein.

Continuing to refer to FIG. 2, the force sensor 15 may be provided for a measurement of contact force between the catheter 12 and tissue 18. For example, the force sensor 15 may be configured to generate an output from which a contact force magnitude and/or force vector may be measured. The contact force may be representative of contact between a portion of the catheter 12 (e.g., the interactive element 17) and tissue 18, in an embodiment. For example, force sensor types such as ultrasound, magnetic, impedance, strain gauge, piezoelectric, or other sensors for detecting force can be used with respect to embodiments of the present disclosure.

The ablation generator 23 may be coupled with the catheter 12 (e.g., electrically coupled with the plurality of interactive elements 17 in FIG. 2) and configured to provide ablation energy to the catheter 12 (e.g., to the plurality of interactive elements 17). For example, the ablation generator 23 may provide an electrical signal at about 450 MHz, in an embodiment. The ablation generator 23 may be further configured to measure an impedance of tissue 18, in an embodiment.

The force sensing system 25 (FIGS. 1 and 2) may be electrically coupled with the force sensor 15 (FIG. 2) for determining a contact force between the catheter 12 and tissue 18. The force sensing system 25 may include a processor 27, a memory 29, and an optical signal source 31, in an embodiment. The optical signal source 31 may be coupled with one or more optical fibers in the catheter 12 and configured to provide an optical signal through the optical fibers. The force sensing system 25 may be further configured to receive the reflected optical signal and calculate a contact force based on the reflected optical signal. In an embodiment, the optical signal source 31 may be configured to generate and transmit optical signals for three optical fibers in the catheter, and the force sensing system 25 may be configured to receive three reflected optical signals and calculate a contact force vector (e.g., magnitude and direction) between the catheter 12 and tissue 18.

The force sensing system 25 and force sensor 15 (FIG. 2) may include technology similar to or the same as that used in the TactiCath^{™} Quartz^{™} Ablation Catheter system, commercially available from St. Jude Medical, Inc. of St. Paul Minnesota. Additionally, or alternatively, the force sensing system 25 and force sensor 15 may include force sensing sensors, systems, and techniques illustrated and/or described in one or more of U.S. Patent Application Publication Nos. 2007/0060847; 2008/0009750; and 2011/0270046, each of which is hereby incorporated by reference in its entirety as though fully set forth herein.

Referring still to Figs. 1 and 2, the mapping and navigation system 30 may be provided to enable a clinician to visualize tissue 16 and to navigate the catheter 14 to and around tissue 16 targeted for diagnosis or therapy, among other functions. Accordingly, the mapping and navigation system 30 may be provided with a variety of functions, including the generation and display of models of tissue, generation and display of electrophysiological (EP) maps of tissue 16, tracking of the catheter, and superimposition of the catheter 14 on a display 34 of one or more maps or models to enable a clinician to view the location of the catheter 14 relative to tissue 16, among other functions. The mapping and navigation system 30 may comprise an ECU 32 and a display 34, with the ECU 32 including a processor 33 and a memory 35, for carrying out the functions described herein and/or other functions.

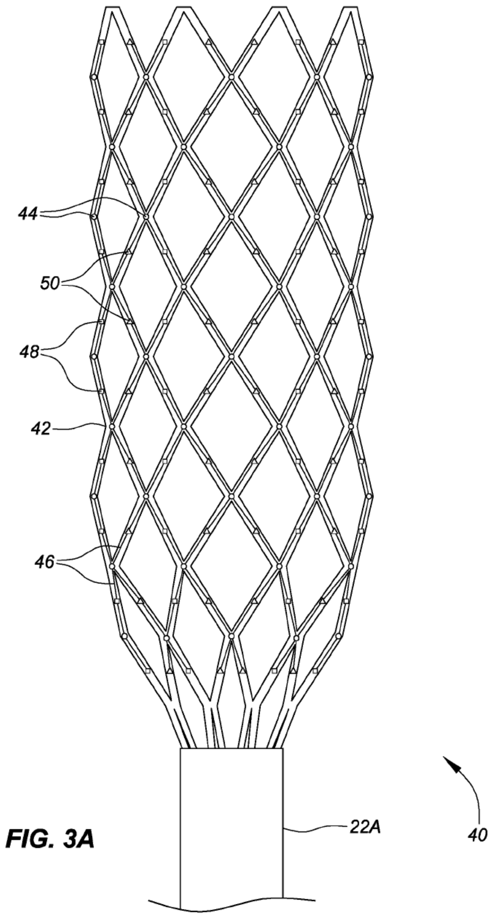

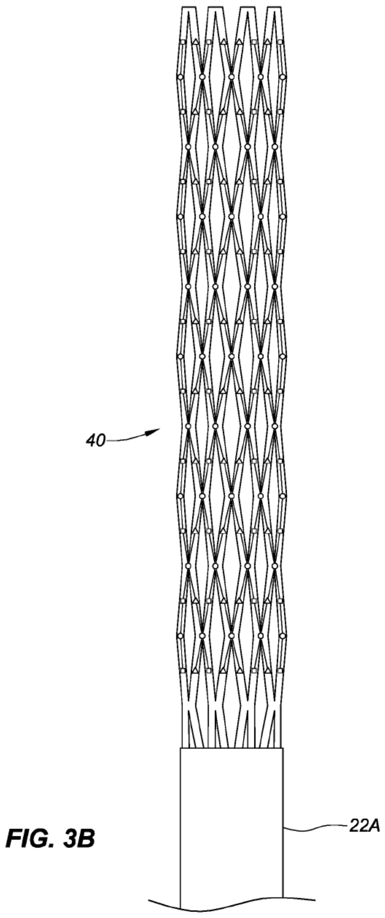

FIG. 3A is a side view of a flexible catheter structure in an expanded state, in accordance with embodiments of the present disclosure. The flexible catheter structure 40 includes an expanded state (shown in FIG. 3A) and a collapsed state (see FIG. 3B). The flexible catheter structure 40 can be in the collapsed state during delivery to an intended therapy site and then be deployed to the expanded state (shown in FIG. 3A) after the flexible catheter structure 40 reaches the intended therapy site. In the collapsed state, the flexible catheter structure 40 can fit into, for example, a catheter or introducer for delivery to the intended therapy site. The flexible catheter structure 40 can be made from a material that retains a shape and permits self-expansion after being collapsed, such as nitinol or other materials that have shape memory. Other suitable materials known in the art of medical stents may also be used. The material can be, for example, braided to form the expandable structure. The flexible catheter structure 40 can be an interlaced support structure (e.g., where various members of the support structure are connected, interwoven, intertwined, intercrossed, or similarly arranged. For example, exemplary braid technologies can be found in the St. Jude Medical AMPLATZER^{™} P.I. Muscular VSD Occluder which can be used for or as part of the expandable structure 42. Additional information regarding the St. Jude Medical AMPLATZER^{™} P.I. Muscular VSD Occluder can be found in, for example, U.S. patent application 6,123,715, is herein incorporated by reference in its entirety.

The flexible catheter structure 40 can be any suitable shape. For example, the flexible catheter structure 40, when expanded, can be cylindrical, conical, elliptical, spherical or other shapes. The collapsed state of the flexible catheter structure 40 can be any suitable shape (e.g., shown in FIG. 3B). For example, the collapsed first position can be cylindrical, to facilitate fitting the expandable structure into the shaft 22A of the medical device 12. In other embodiments, the flexible catheter structure 40 2 in the collapsed state can fit into, for example, a catheter or introducer for delivery to an intended therapy site. In some embodiments, the expandable structure can extend distally about a longitudinal axis from a distal end of the shaft 22A. In transition from the collapsed state to the expanded state, the flexible catheter structure 40 can be radially expanded outwardly from the longitudinal axis from the collapsed state to the expanded state.

The expansion of the flexible catheter structure 40 as it is deployed can provide sufficient expansion force to cause the sensors 44 to contact the tissue at the therapy site. The expansion force may be varied by controlling the expansion of the flexible catheter structure 40. For example, the flexible catheter structure 40 may permit variable expansion forces following an initial set pre-form shape to facilitate entering and engaging pulmonary veins. The expansion of the flexible catheter structure 40 can be measured using an appropriate sensor such as, for example, a force sensor, a strain gauge or strain sensor.

In some embodiments, the flexible catheter structure 40 can include a balloon or similar device (not shown) that can be expanded using one or more gases (e.g., air) or a fluid (e.g., water). For example, the balloon can be expanded when fluid or gas is added to the interior of the balloon to transform the balloon from a contacted state to an expanded state. In some embodiment, the expansion of the balloon can, in turn, can cause the flexible catheter structure 40 to be expanded to cause the sensors 44 to contact the tissue at the therapy site.

Returning to FIG. 3A, the flexible catheter structure 40 can include one or more sensors 44. The sensors 44 can include, for example, electrodes, thermocouples, force sensors (to register, for example, tissue contact and/or total force exerted on tissue), strain gauges, strain sensors, position sensors, biosensors (e.g., capable of converting a biological response to an electrical signal), diagnostic sensors, therapy sensors, chemical sensors (e.g., capable of delivery and or monitoring of drugs/chemicals, etc.), light-emitting sensors, acoustic sensors, ultrasound sensors, energy receiving and/or measuring sensors, a magnetic coil or sensor, thermoelectric elements, or other sensors. In some embodiments, the one or more sensors 44 can be combined with another sensor and/or an energy delivery element into one element, also called a "interactive element." The interactive elements can permit tighter spacing of the elements (e.g., instead of alternating between sensor and energy delivery elements which could lead to gaps in coverage). The combinations of sensors, which can be included in the interactive element, can include any sensor (some examples listed above including an energy delivery element, a thermocouple, a force sensor, a strain gauge, a strain sensor, a position sensor, and a bio-sensor) in any combination (two sensors, three sensors, four sensors, etc.).

Some embodiments can include, for example, three sensors and/or elements combined to form a tri mode element or other multiples of sensors and/or elements combined to form a "multiple mode element." The interactive elements, can be dual mode elements, tri mode elements, and/or multiple mode elements and can be used with, for example, any of the embodiments described herein. In some embodiments, the flexible catheter structure 40 can include, in addition to one or more sensors 44, a plurality of interactive elements 48 or sensors 44. The one or more sensors 44 can be attached to the flexible catheter structure 40 using any suitable method (e.g., adhesive) including additive manufacturing methods (e.g., direct deposition).

In some embodiments, the flexible catheter structure 40 can also include a substrate (not shown). The substrate can be any suitable material including, for example, a polymer or a metal, and the substrate can be in any suitable configuration (e.g., a film, a mesh, a braid, etc.). The substrate can be flexible. The substrate can be connected or attached to one or more locations of the flexible catheter structure 40. The substrate can be attached to the flexible catheter structure 40 by any suitable method including, for example, adhesive. The one or more sensors 44 and/or the plurality of interactive elements 48 can be located on the substrate that is connected to the flexible catheter structure 40. In some embodiments, the substrate can be separate from the flexible catheter structure 40. The sensors 44 and/or interactive elements 48 can be electrically connected (e.g., a plurality of conductive electrical traces 46, wires, etc.) to a power supply, controller, medical positioning system 14 or other device used to generate a signal.

In some embodiments the flexible catheter structure 40 can include one or more energy delivery devices 50. The energy delivery devices 50 can be attached to the flexible catheter structure 40 using any suitable method. This includes, for example, printing the energy delivery devices 50 directly onto the flexible catheter structure 40 (e.g., an ink jet process). The energy delivery devices 50 can be electrically connected (e.g., the plurality of conductive electrical traces 46, wires, etc.) to a power supply, controller, medical positioning system 14 or other device used to generate a signal.

The various sensors and elements (e.g., sensors 44, interactive elements 48, energy delivery devices 50, etc.) can be arranged in various patterns on the flexible catheter structure 40 or the substrate. For example, the sensors and/or elements can be evenly distributed with equal spacing (e.g., a similar density) or they can have different spacing or varying concentrations (e.g., a different or variable density) of sensors/elements in locations on the flexible catheter structure 40 or substrate. In some embodiments that include the flexible catheter structure 40 and the substrate there can be sensors and/or elements on both.

The energy delivery devices 50 can be located at any suitable location on the expandable structure. For example, the energy delivery devices 50 can be arranged in a pattern (symmetrical or asymmetrical) to provide desired distribution and coverage during use. For example, a symmetrical pattern can provide symmetrical coverage of the intended therapy site and an asymmetrical pattern can target certain areas of the intended therapy site.

FIG. 3A shows the sensors 44, the interactive element 48, and the energy delivery devices 50 and various locations on the flexible catheter structure. The exemplary arrangement shown is not meant to be limiting as the various element (e.g., the sensors 44, the interactive element 48, and the energy delivery devices 50) can be located at any of the locations shown (e.g., the interactive element 48 can be located where some and/or all of the sensors 44 are shown in FIG. 2, the energy delivery devices 50 can be located where some and/or all of the interactive elements 48 are located, etc.).

The flexible catheter structure 40 can be delivered to the desired location of the body using the shaft 22A. The flexible catheter structure 40 can be located inside the end of the shaft 22A. Using, for example, a balloon, pull wires or similar methods, the flexible catheter structure 40 can be protracted with respect to the shaft 22A and/or the shaft 22A can be retracted with respect to the flexible catheter structure 40, causing the flexible catheter structure 40 to be exposed as the structure is distally advanced beyond the end of the shaft 22A.

FIG. 3B is a side view of the flexible catheter structure from FIG. 2A in a contracted state, in accordance with embodiments of the present disclosure. The flexible catheter structure 40 can have a collapsed state of the flexible catheter structure 40 allows for easier movement of the expandable structure when delivering the expandable structure into various locations of a patient (e.g., intended therapy sites). The collapsed state also assists with deploying the flexible catheter structure 40 from the shaft 22A and storage of the flexible catheter structure 40 within the shaft 22. For example, the flexible catheter structure 40 fits inside the shaft 22A during delivery. During deployment the shaft 22A can be retracted and/or the flexible catheter structure 40 can be protracted to permit the flexible catheter structure 40 to be expanded (e.g., to the expanded state shown in FIG. 3A) using one of the methods described above or other suitable method.

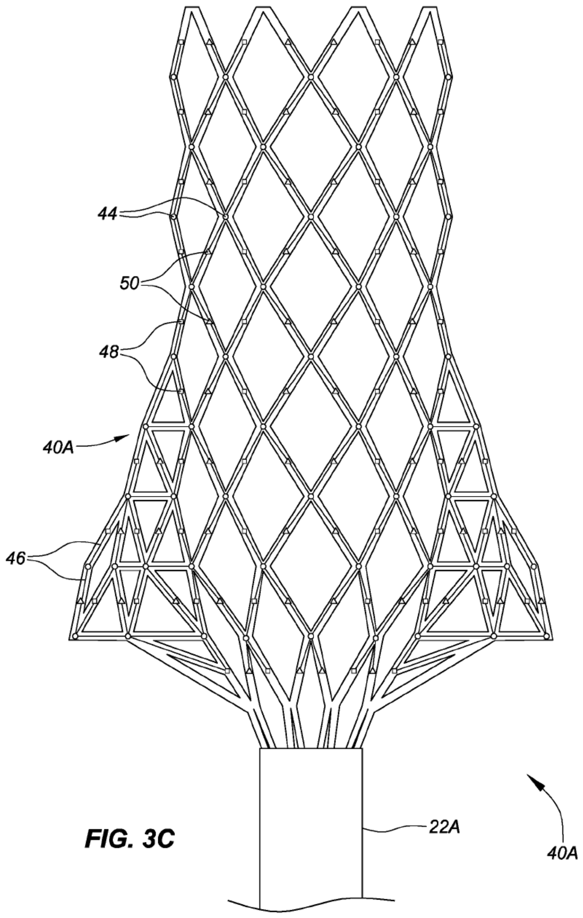

FIG. 3C is a side view of a flexible catheter structure similar to FIG. 3A with additional annular structure, in accordance with embodiments of the present disclosure. The flexible catheter structure 40A can be similar to the flexible catheter structure 40 shown in FIG. 3A and described above. The flexible catheter structure 40A can include additional elements to create the shape of the structure as shown in FIG. 3C. The flexible catheter structure 40A can include a bulbous annular shape and/or "wings" as shown in FIG. 3C to facilitate contact between the flexible catheter structure 40A and tissue (e.g., contact with the PV, including tissue inside the PV, proximate an opening to the PV (e.g., the antral wall), etc.). Similar to the description above for FIGS. 3A-B, the flexible catheter structure 40A can be deployed from the shaft 22A (e.g., to an expanded state) and collapsed (e.g., to a collapsed state) to fit inside the shaft 22A during deployment.

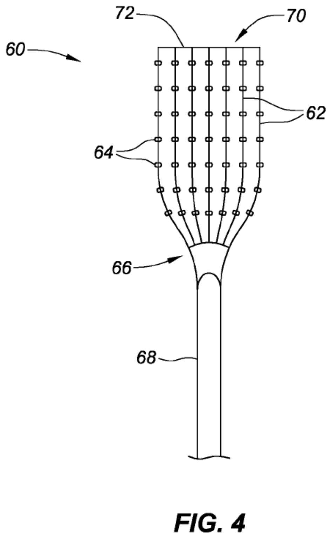

FIG. 4 is a side view of another embodiment of a flexible catheter structure, in accordance with embodiments of the present disclosure. A flexible catheter structure 60 can include a plurality of flexible wires (or struts) 62 to support a plurality of interactive elements 64. For example, the expandable structure can include seven struts as shown in Fig. 4. However, some embodiments could have a fewer or greater number of struts. The interactive elements 64 are described in greater detail above. The flexible wires or struts 62 can be made out of any suitable material that permits the desired shape including nitinol and other materials that have shape memory.

The flexible catheter structure 60 can be, in some embodiments, naturally biased to be in an expanded state. In another embodiment, the flexible catheter structure 60 can be biased to be in a collapsed state. The flexible catheter structure 60 can have any suitable number of flexible wires or struts 62. The flexible catheter structure 60 can be supported on the proximal end 66 where it is attached to a shaft 68. In some embodiments, the distal end 70 can include an end support 72. The end support 72 can, for example, provide support for various end shapes of the flexible catheter structure 60 at the distal end 70. The end support 72 can also connect the plurality of wires or struts 62. In some embodiments, the distal end 70 can be open (e.g., a hoop, a circle, an oval, etc.). In other embodiments, the distal end 70 can be closed (e.g., the distal end 70 has additional flexible catheter structure 60 to form a surface (e.g., flat, convex, concave, etc.)) or reduce the size of the opening at the distal end 70. In other embodiments, the distal end 70 can be a combination of the two. The embodiment can be similar to the St. Jude Medical device HD EnSite^{™} Array^{™} Catheter.

In some embodiments, the flexible catheter structure 60 can include a flexible substrate (not shown) in addition to the plurality of wires (or struts) 62 described above. The flexible substrate can include one or more interactive elements similar to other embodiments described herein.

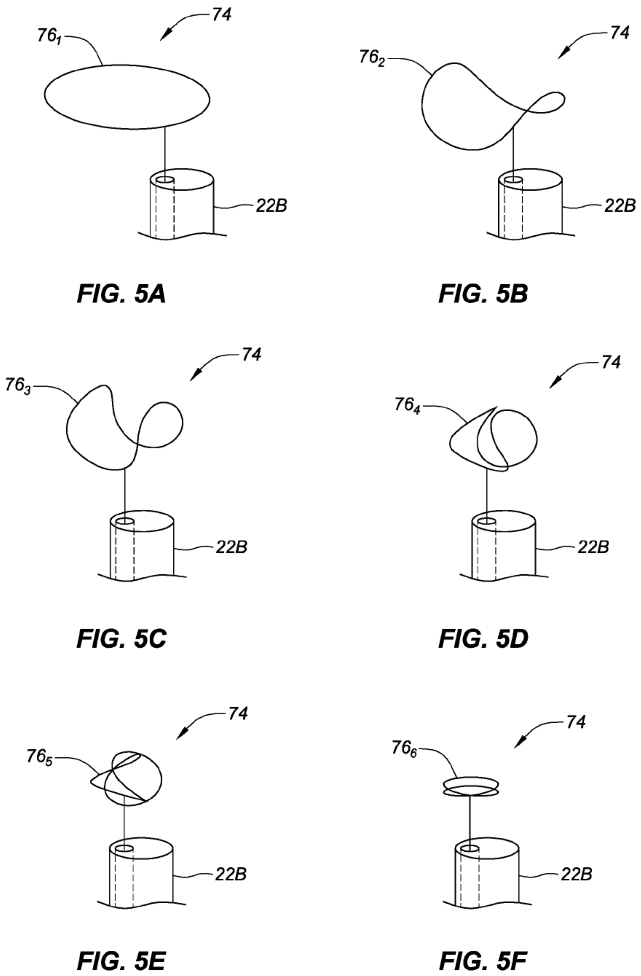

FIGS. 5A-F are isometric side and distal end views of exemplary configurations of a flexible catheter structure in various states of expansion, in accordance with embodiments of the present disclosure. Fig. 5A is an isometric side and distal end view of an flexible catheter structure 74 in a first expanded state 76₁ (e.g., deployed state). As depicted, the flexible catheter structure 74 can include a single element connected at either end to form a circular structure. The flexible catheter structure 74 can be deployed from a shaft 22B (e.g., similar to FIGS. 3A-C). In some embodiments, the flexible catheter structure 74 can form other shapes, such as a triangle, oval, square, etc. Figure 5B is an isometric side and distal end view of the flexible catheter structure 74 in a second expanded state 76₂. In contrast to Fig. 5A, opposite sides of the circular structure have been folded up to form a saddle shape (e.g., hyperbolic paraboloid). Figs. 5C and 5D depict the opposite sides of the saddle of the flexible catheter structure 74, in states 76₃ and 76₄ being further disposed in an upward fashion and towards one another. Figure 5E depicts further changes in the shape of the flexible catheter structure74 to continue the "folding" (e.g., collapsing) process. Figure 5F depicts an isometric side and distal end view of the flexible catheter structure 74 in a sixth state 76₆ (e.g., stored state) where the circular structure has been configured to be smaller than the first expanded state. The sixth state allows for easier movement of the flexible catheter structure 74 when delivering the flexible catheter structure74 into various locations of a patient (e.g., intended therapy sites). This can be achieved by permitting the flexible catheter structure 74 to fit into a catheter, introducer, or other similar device.

The flexible catheter structure 74 can have a support structure that is configured to fold up or collapse into a smaller arrangement/configuration to permit the flexible catheter structure 74 to fit into a desired size and/or shape. For example, the flexible catheter structure 74 can be in a stored state 76₆ to be used while the expandable structure is, for example, stored inside a shaft (e.g., the shaft 22B) or other similar delivery device or attached to the outside of a shaft. After the shaft is maneuvered to the desired location of the body (e.g., inside a heart) the flexible catheter structure 74 can be expanded. For example, the flexible catheter structure 74, when in state 76₆ can be sized to fit into the shaft 22B while being maneuvered to a location (e.g., a heart) and then deployed as described herein. For example, pull wires or other suitable mechanisms can be used to "unfold" or expand the flexible catheter structure 74 from the sixth state 76₆ depicted in Fig. 5F to the first expanded state 76₁ depicted in Fig. 5A.

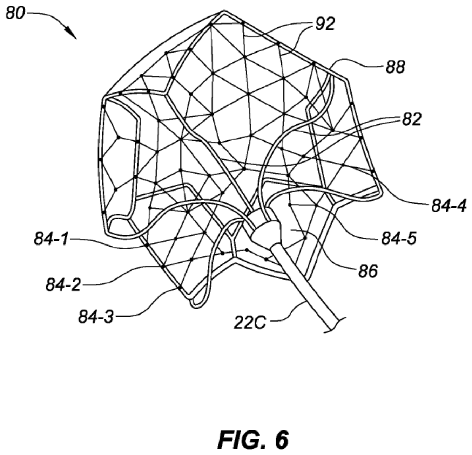

FIG. 6 is an isometric side and proximal end view of an exemplary embodiment of an flexible catheter structure, in accordance with embodiments of the present disclosure. The flexible catheter structure 80 can have a support structure that is configured to fold up or collapse into a smaller arrangement to permit the flexible catheter structure 80 to fit into a desired size and/or shape. For example, the flexible catheter structure 80 can be in a first collapsed state (not shown), allowing the flexible catheter structure 80 to be stored, for example, inside a shaft 22C or other similar delivery device, or attached to the outside of the shaft 22C. After the shaft 22C is maneuvered to the desired location (e.g., inside a heart) of the body 16 the flexible catheter structure 80 can be expanded to a second expanded state (e.g., deployed), as depicted in Fig. 6. For example, one or more pull wires or other suitable mechanisms can be used to "unfold" or expand the flexible catheter structure 80 from the first collapsed state to the second expanded state depicted in Fig. 6. In some embodiments, the flexible catheter structure can be dome shaped in the second expanded state, as depicted. However, in some embodiments, the flexible catheter structure can be expanded into other shapes in the second expanded state, such as conical, etc.

In the embodiment shown in FIG. 6, the flexible catheter structure 80 can include a plurality of struts 82 and a flexible substrate 84. In other embodiments, the flexible catheter structure 80 can be used without a flexible substrate similar to descriptions above for FIG. 3A. The flexible substrate 82 can be a single element that covers all desired parts (e.g., the struts 82) of the flexible catheter structure 80 or multiple smaller flexible substrates can be used to cover different parts of the flexible catheter structure 80 (e.g., between various struts 82). The flexible substrate 84 can be, for example, stretched over an inner surface or outer surface of the flexible catheter structure 80 (e.g., tented) or located between struts 82 of the flexible catheter structure 80.

The flexible substrate 84 can include a plurality of interactive elements 86 located on the struts 82 of the flexible catheter structure 80 and/or on the flexible substrate 84. The plurality of interactive elements 86 can be similar to other interactive elements described herein. The plurality of interactive elements 86 can be located at any location on the flexible catheter structure 80 similar to descriptions above for FIG. 3A. The expandable structure 80 can have a first state where the flexible catheter structure 80 is expanded as shown in FIG. 6. The first expanded state shown in FIG. 6 can cause at least one of the interactive elements 86 to be in contact with tissue at a therapy site (e.g., the heart). The flexible catheter structure 80 can also have a second state where the expandable structure 80 is collapsed during delivery to an intended therapy site (not shown). The flexible catheter structure 80 in the collapsed state can fit into, for example, a catheter or introducer for delivery to the intended therapy site.

The flexible catheter structure 80 can include a plurality of interactive elements 86. The interactive elements 84 can be electrically connected (e.g., a plurality of conductive electrical traces 88, wires, etc.) to, for example, a power supply, controller, medical positioning system 14 or other device used to generate a signal. The interactive elements 86 can allow for, for example, active sensing in a distal location in the PV, which can provide acute measurement of effectiveness in one device. The measurement of effectiveness can include, for example, measuring electrical activity, resistance, reactance, impedance, tissue contact, tissue force, temperature, energy, power, time, etc.

The interactive elements 86 can incorporate temperature or other types of sensors, which can be used to obtain data at a therapy delivery (e.g., ablation) location. The interactive elements 86 can include an energy delivery device, as described above, that provides ablation energy for ablating tissue to create lesions. The interactive elements 86 can be spaced in manner to address gap closure (e.g., reducing and/or eliminating gaps between ablation lesions) in a mono-polar arrangement (e.g., using individual lesion growth and close spacing of interactive elements), a bi-polar arrangement (e.g., between electrodes), or a combination of both. The spacing of the interactive elements 86 can be constant (e.g., equal distance) depending on the design and shape of the flexible catheter structure 80.

For example, if the interactive elements 86 are along the same strut 82 or structure of the flexible catheter structure 80, the spacing between each of the interactive elements 86 along the strut 82 can be fixed. The spacing between interactive elements 86 on different struts 82 or structures (e.g., the interactive elements 86 on the flexible substrate 84) of the flexible catheter structure 80 can vary as the amount of expansion varies. The interactive elements 86 can be selectively activated (e.g., one or more of the interactive elements are activated while other ones of the interactive elements remain inactivated) by the user. Various patterns can be generated as different combinations of interactive elements 86 are selected for use. The variation in patterns can, for example, permit the user to address different anatomical sizes in different locations of the body and/or or different patients. Another exemplary use of the variation in patterns can be to create different patterns of lesions. In some embodiments, the interactive elements 86 can include additional elements or sensors. For example, more than one sensor may be included in the element along with an energy delivery element (e.g., a thermocouple and a tissue contact sensor).

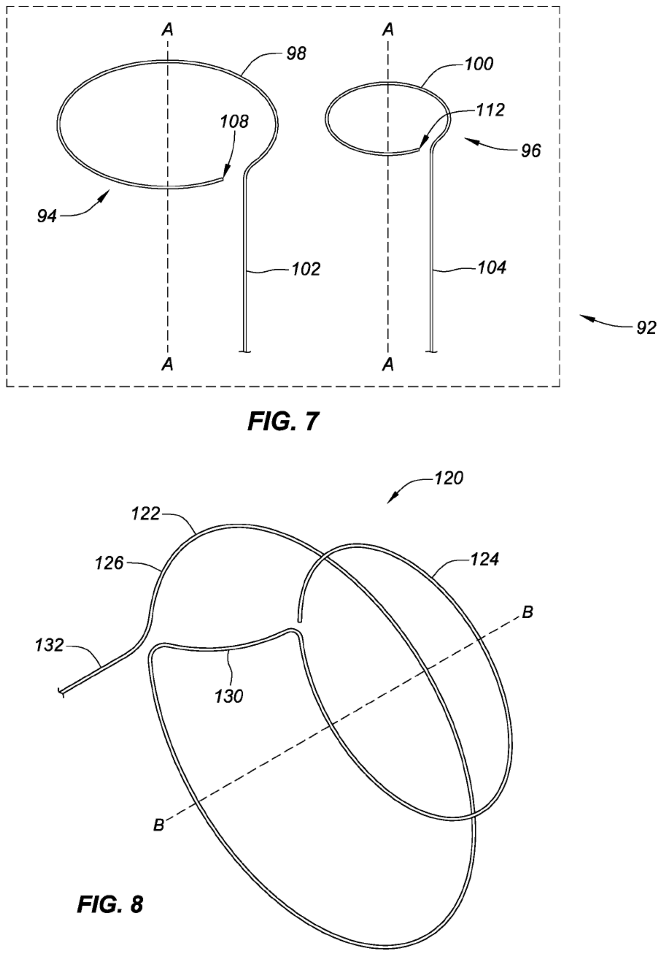

FIG. 7 is an isometric side and distal end view a flexible catheter structure comprising a first shaping element and a second shaping element, shown juxtaposed prior to use during a medical procedure, in accordance with embodiments of the present disclosure. The flexible catheter structure 92 can comprise a first shaping element 94 and a second shaping element 96. The first shaping element 94 can include a first shaping hoop 98 and the second shaping element 96 can include a second shaping hoop 100 that can each be circular in shape. The first and second shaping hoops 98 and 100 can be other shapes (elliptical, rectangular, square, etc.). In some embodiments, the first and second shaping hoops 98, 100 can be each be of a different shape. For example, the first shaping hoop 98 can be circular and the second shaping hoop 100 can be elliptical, etc. The first shaping element 94 and second shaping element 96 can include a first and second connecting sections 102 and 104 (e.g., support structures) that are connected to the first and second shaping hoops 98 and 100, respectively.

The a first and second connecting sections 102 and 104 can be connected to pull wires or other similar devices to facilitate deployment of the first shaping element 94 and second shaping element 96 at various locations in a body. In some embodiments, the a first and second connecting sections 102 and 104 and first and second shaping hoops 98 and 100 associated with each of the first and second shaping elements 94 and 96, respectively, can be formed from a unitary piece of material. For example, the first shaping hoop 98 can be formed from a unitary piece of material, which can include a distal end 108 and/or the second shaping hoop 100 can be formed from a unitary piece of material, which can include a distal end 112.

The first and second shaping hoops 98 and 100 may not be complete circles. For example, the first and/or second shaping hoops 98, 100 can be open ended, as depicted in Fig. 7. Accordingly, the distal ends 108 and 112 may be unattached. In some embodiments, the first and/or second catheter end hoops can be closed ended, where the distal ends are connected to form a closed hoop (e.g., the distal ends 108 and 112 are coupled with a portion of the first or second connecting sections 102 or 104).

Each of the first and second shaping elements 94 and 96 can be formed, for example, from an elongate element that includes a proximal portion and a distal portion. In some embodiments, the proximal portion can be the first connecting section 102 and/or the second connecting section 104 that are parallel to an axis defined by the line AA In some embodiments, the distal portion can be formed by the first shaping hoop 98 and/or the second shaping hoop 100, where the plane of the hoop forms a particular angle (e.g., perpendicular) with the axis defined by the line AA

For example, the elongate element can transition from a straight proximal portion into a hooped distal portion (e.g., from the first connecting section 102 to the first shaping hoop 98). The first shaping element 94 and second shaping element 96 may be a complete circle in some embodiments (e.g., the distal ends 108, 112 of the elongate elements of the first catheter end hoop 98 and second catheter end hoop 100 can be connected to the a first connecting section 102, 104 and the hoops 98, 100). The first shaping element 94 and second shaping element 96 can be formed from separate elongate elements formed from a material that has shape memory (e.g., nitinol, stainless steel, and/or polymers) to allow the first and second shaping elements 94 and 96 to assume a naturally biased shape upon being deployed at a location in a body (e.g., therapy site) similar to the descriptions above. In other embodiments, one or more pull wires can be attached to one or more locations on either the first or second shaping hoops 98, 100 or the one or more pull wires can be attached to both the first and second shaping hoops 98, 100. This can allow for further manipulation of the first and/or second shaping elements 98, 100 for positioning to, for example, increase contact between the shaping elements and tissue.

FIG. 8 depicts an isometric side and distal end view of a flexible catheter structure that can include that can include an alternative shaping element comprising, a plurality of shaping hoops that are connected in this embodiment, where the plurality of shaping hoops comprises a first or proximal shaping hoop and a second or distal shaping hoop, in accordance with embodiments of the present disclosure. In contrast to Fig. 7, a single elongate element can be used to form a flexible catheter structure that comprises a shaping element 120. The shaping element 120 can include a first shaping hoop 122 and a second shaping hoop 124. In some embodiments, the shaping element 120 can include additional shaping hoops (e.g., a third shaping hoop, a fourth shaping hoop, etc.). Additional shaping hoops can be used to, for example, change the profile of the shape created to allow for better contact with variations in tissue shapes/configurations.. As described herein, the additional shaping hoops can similarly be connected to, for example, one or more pull wires or other similar devices that allow manipulation of the shaping hoops (e.g., longitudinal movement of the shaping hoops, changing the shape of the shaping hoops, etc.).

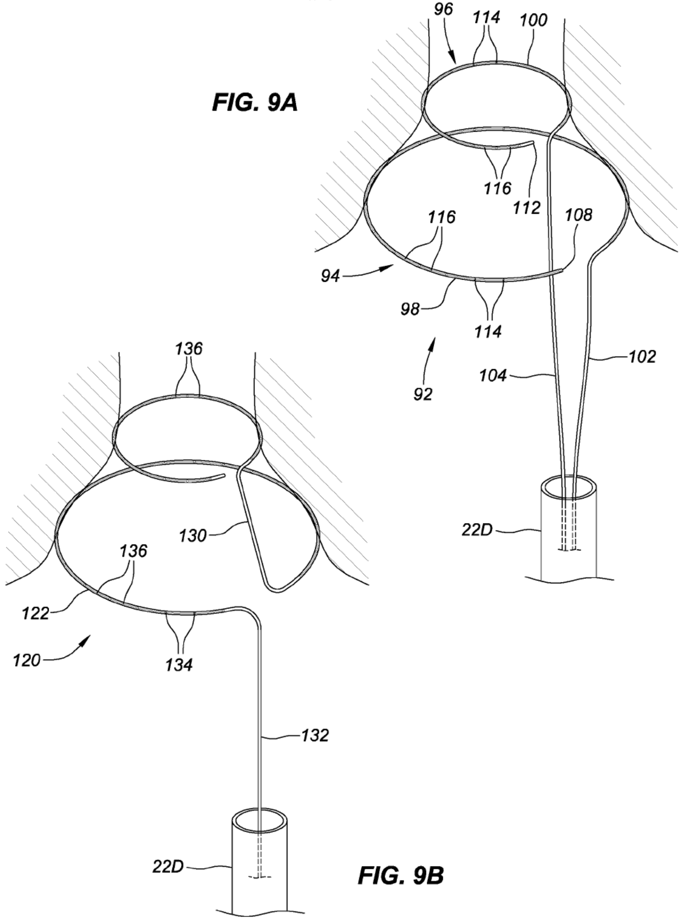

The first and second shaping hoops 122 and 124 can include a plurality of interactive elements 134 (shown in FIG. 9B). The plurality of interactive elements can be mounted on the first and second shaping hoops 122 and 124 using any suitable method (e.g., adhesive, etc.). The plurality of interactive elements 134 can be connected with a plurality of conductive electrical traces 136. The conductive electrical traces 136 can be electrically connected (e.g., a plurality of conductive electrical traces, wires, etc.) to a power supply, controller, medical positioning system 14 or other device used to generate a signal.

In some embodiments, the first shaping hoop 122 can have a larger radius than the second shaping hoop 124 and can be located proximally with respect to the second shaping hoop 124. A hoop interconnecting section 130 (e.g., a support structure) can connect the first and second shaping hoops 122 and 124. The hoop interconnecting section 130 can be any suitable length to achieve the desired space between the first and second shaping hoops 122 and 124. Similar to FIG. 6 above, the first shaping hoop 122 can be connected to a connecting section 132 (e.g., a support structure) that can be connected to pull wires or other similar devices to facilitate deployment of the single elongate element that can form the first shaping hoop 122 and second shaping hoop 124 at various locations in a body 16 (e.g., the heart).

The first shaping hoop 122 and the second shaping hoop 124 can be aligned so that the hoops 122 and 124 of each shaping hoop is centered about an axis defined by the line BB. The connective section 132 can also have a portion aligned with the axis defined by the line BB, but offset from the axis. The first shaping hoop 122 and second shaping hoop 124 may be formed by any suitable method including pre-formed heat set as described above.

The first shaping hoop 122 and second shaping hoop 124 can be formed from wire or polymer or other suitable material. The material used for the first shaping hoop 122 and second shaping hoop 124 can be sufficiently rigid to "re-model" the PV in some embodiments (e.g., with a material that is sufficiently rigid at a particular diameter for the shaping hoops). The shaping element 120 can be maneuvered so that the first shaping hoop 122 and the second shaping hoop 124 can be positioned at a location in the body (e.g., the PV or any other surface structural shape). The sizes (e.g., the diameter of the first shaping hoop 122 and the second shaping hoop 124 can be specified so that they can be slightly larger than the anticipated diameter of the location in the PV). The first shaping hoop 122 and the second shaping hoop 124 can be placed so that they are in contact with portions of the PV. This contact can cause what is called "re-modeling" of the PV. The re-modeling of the PV can occur when the PV temporarily takes the shape of the first shaping hoop 122 and second shaping hoop 124. The re-modeling of the PV can increase contact between tissue and the flexible catheter structure (e.g., the shaping element 120).

In some embodiments, the first shaping hoop 122 and the second shaping hoop 124 can have variable sizes changes and/or adjustments to the shape due to, for example, pull wires or other similar devices. The adjustability of the first shaping hoop 122 and the second shaping hoop 124 can allow for increased contact between the first shaping hoop 122 and the second shaping hoop 124 and tissue.

FIG. 9A is an isometric side and distal end view of a flexible catheter support structure that includes the first shaping element and the second shaping element, as shown in FIG. 7, being located in a pulmonary vein (PV), in accordance with embodiments of the present disclosure. The first shaping element 94 (lower, or proximal) is sized to fit into the wide antral portion of the PV. The second shaping element 96 (upper, or distal) is sized to fit further into the sleeve of the PV past the wider antral portion near the opening of the PV. The first shaping element 94 and the second shaping element 96 can include diameters ranging between, for example, 12-33 mm. The first and second shaping elements 94 and 96 can be maneuvered adjacent to the PV using any suitable method and the first shaping element 94 and the second shaping element 96 can be maneuvered to a location of the PV. The location of the PV can be, for example, where the PV is approximately the same size (diameter) as the first shaping element 94 and second shaping element 96 (e.g., near the opening of the PV). In some embodiments, the first shaping element 94 and second shaping element 96 can be separately placed (e.g., each catheter end shape is deployed with, for example, a separate catheter or introducer) at a location in the PV. The first shaping element 94 and second shaping element 96 can also be separately moved (e.g., the first shaping element 94 can be moved independently of the second shaping element 96 and vice versa) to allow for better contact between the first shaping element 94 and second shaping element 96 and tissue.

Similar to above, the first and second shaping elements 94 and 96 can include a plurality of interactive elements 114 (shown in FIG. 9A). The plurality of interactive elements can be mounted on the first and second shaping elements 94 and 96 using any suitable method (e.g., adhesive, etc.). The plurality of interactive elements 114 can be connected with a plurality of conductive electrical traces 116. In some embodiments, the first and second shaping elements 94 and 96 can include a plurality of electrodes (e.g., ring electrodes) (not shown). The plurality of ring electrodes can be located on the first and second shaping elements 94 and 96 can be connected (e.g., electrically and mechanically) in a manner similar to the interactive elements. The ring electrodes can be located at any suitable location on the flexible catheter structure, including, for example, the locations of the sensors 44, interactive elements 114, and/or energy delivery devices 50 as shown in FIG. 3A or interactive elements 114, sensors 148 described herein). The conductive electrical traces 116 can be electrically connected (e.g., a plurality of conductive electrical traces, wires, etc.) to a power supply, controller, medical positioning system 14 or other device used to, for example, generate, amplify, receive, and/or process a signal.

As a result of the first shaping element 94 and second shaping element 96 being positioned in the PV, the interactive elements 114 can contact the tissue of the PV. The first shaping element 94 and second shaping element 96 can be placed so that they re-model the PV (e.g., stretch the tissue at that location of the PV, but only in a temporary manner). The re-modeling of the PV can be caused, for example, by the compliancy of the PV compared to the structure of the first shaping element 94 and the second shaping element 96.

Similar to FIGS. 3A-C above, the first shaping element 94 and second shaping element 96 can fit inside a shaft 22D during delivery. During deployment the shaft 22D can be retracted and/or the first shaping element 94 and second shaping element 96 can be protracted to permit the first shaping element 94 and second shaping element 96 to be expanded using one of the methods described above or other suitable method.

FIG. 9B is an isometric side and distal end view of a flexible catheter support structure that depicts a single elongate element that forms a first shaping hoop and a second shaping hoop, similar to FIG. 8, in a PV, in accordance with embodiments of the present disclosure. Similar to above, the first shaping hoop 122 can be sized to fit into the wide antral portion of the PV. The second shaping hoop 124 can be sized (e.g., smaller diameter) to fit further into the sleeve of the PV past the wider antral portion near the opening of the PV. Similar to FIGS. 5 and 8A above, a plurality of interactive elements 134 can be mounted to the first and second shaping hoops 122 and 124.

In some embodiments, the first shaping hoop can be sized to be wider than an opening of the PV (not shown). For example, the first shaping hoop can be larger than the embodiment shown with the shaping hoop 122 in FIG. 9B, allowing the first shaping hoop to contact tissue proximate the PV opening (e.g., resting against an antral wall). In some embodiments, the second shaping hoop can be sized to fit into various locations of the PV. For example, with a larger first shaping hoop as described above the second shaping hoop can be sized similar to the first shaping hoop 122 shown in FIG. 9B, or smaller than the second shaping hoop 124 shown in FIG. 9B (e.g., to all the second shaping hoop to fit into smaller portions of the PV and/or other locations).

Similar to FIG. 9A above, as a result of the first shaping hoop 122 and second shaping hoop 124 being positioned proximate the PV, the interactive elements 134 can contact the tissue of the PV. The first catheter shaping hoop 122 and second catheter shaping hoop 124 can be placed so that they re-model the PV (e.g., stretch the tissue at that location of the PV, but only in a temporary manner). The re-modeling of the PV caused by the compliancy of the PV compared to the structure of the first shaping hoop 122 and second shaping hoop 124.

Similar to FIGS. 3A-C and 9A above, the first shaping element 94 and second shaping element 96 can fit inside the shaft 22D during delivery. During deployment the shaft 22D can be retracted and/or the first shaping element 94 and second shaping element 96 can be protracted to permit the first shaping element 94 and second shaping element 96 to be expanded using one of the methods described above or other suitable method.

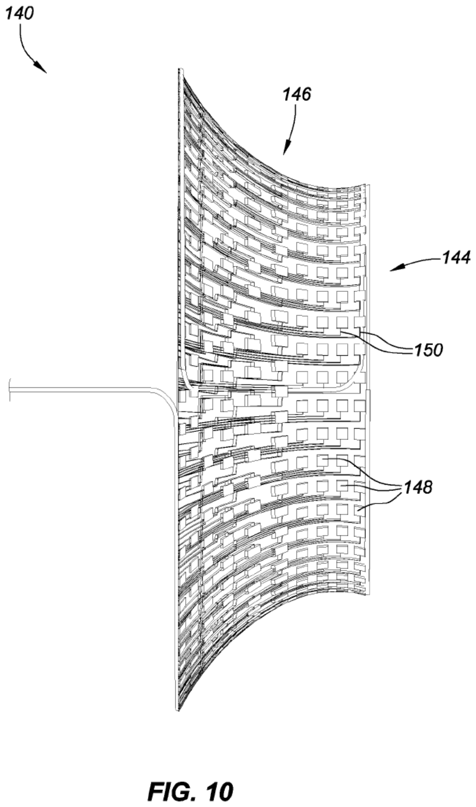

FIG. 10 is an isometric side view of a flexible catheter structure comprising an anatomically configured tissue shaping element that includes a first shaping hoop and a second shaping hoop together with a support structure (flexible substrate) extending there between, , in accordance with embodiments of the present disclosure. As described in FIGS. 8 and 9B above, an anatomically configured tissue shaping element 140 can be used to form a first shaping hoop 142 and a second shaping hoop 144. A flexible substrate 146 can be mounted between the first and second shaping hoops 142 and 144. The flexible substrate 146 can have a plurality of interactive sensors 148 mounted to it. The plurality of interactive sensors 148 can be connected to a plurality of conductive electrical traces 150. The plurality of conductive electrical traces 150 can be connected to a power supply, controller, medical positioning system 14 or other device used to, for example, generate, amplify, receive, and/or process a signal.

The plurality of conductive electrical traces 150 can connect the plurality of interactive elements 148 with separate wires (shown in FIG. 10). In some embodiments, groups of interactive elements can be connected with one conductive electrical trace to facilitate larger numbers of interactive elements combined with small sizes of catheters. In some embodiments, different interactive elements can be controlled using, for example, varying frequencies of transmission along a common conductive electrical trace. Any sensors (e.g., thermocouples) can be wired in a similar arrangement.

In an embodiment shown in FIG. 10, the anatomically configured ablation tissue shaping element 140 can be shaped, to fit a location proximal the PV. For example, FIG. 10 shows the first shaping hoop 142 and the second shaping hoop 144 where each can be sized to fit into a location of the PV (e.g., the first shaping hoop 142 can fit a location more proximal in the opening of the PV and the second shaping hoop 144 can fit a location more distal in the PV). The flexible substrate 146 can be flared or curved between the first shaping hoop 142 and the second shaping hoop 144 to approximately match the corresponding contour or shape of the PV where the first shaping hoop 142 and the second shaping hoop 144 are placed. In other embodiments, the contour or shape of the flexible substrate 146 can be adjusted by changing the shape of the first shaping hoop 142 and/or the second shaping hoop 144. The adjustability can be achieved by, for example, one or more pull wires connected at one or more locations on one or both of the first shaping hoop 142 and/or the second shaping hoop 144.

As shown in the embodiment depicted in FIG. 10, the plurality of interactive sensors 148 can be located on the flexible substrate 146. The plurality of interactive sensors 148 can be arranged, for example, in a pattern with equal spacing between all of the interactive sensors 148. In other embodiments, the interactive sensors 148 can have varying spacing. For example, the varying spacing can include closer spacing near the first shaping hoop 142 (e.g., less distance between the interactive sensors 148, greater density of interactive sensors 148, etc.) and farther spacing near the second shaping hoop 144 (e.g., greater distance between the interactive sensors 148, lower density of interactive sensors 148, etc.).

Similar to FIG. 9B above, in some embodiments, the first shaping hoop can be sized to be wider than an opening of the PV (not shown). For example, the first shaping hoop can be larger than the embodiment shown with the shaping hoop 122 in FIG. 9B, allowing the first shaping hoop to contact tissue proximate the PV opening (e.g., resting against an antral wall). In some embodiments, the second shaping hoop can be sized to fit into various locations of the PV. For example, with a larger first shaping hoop as described above the second shaping hoop can be sized similar to the first shaping hoop 122 shown in FIG. 9B, or smaller than the second shaping hoop 124 shown in FIG. 9B (e.g., to all the second shaping hoop to fit into smaller portions of the PV and/or other locations).

The variations in hoop size described above can allow the flexible substrate 146 to contact various portions of tissue. For example, in embodiments where the first shaping hoop is be larger than the embodiment shown with the shaping hoop 122 in FIG. 9B, the flexible substrate 146 can contact tissue of an antral wall proximate the PV. The flexible structure 146 can conform to a contour of the tissue allowing contact between the plurality of interactive elements 148 and/or other sensors coupled with the flexible substrate 146. With the larger first shaping hoop, mapping and/or therapy (e.g., ablation of tissue) can be applied at any point between the first and the second shaping hoops (e.g., from areas proximate the PV opening to areas into the PV beyond what is shown in FIGS. 9A-B).

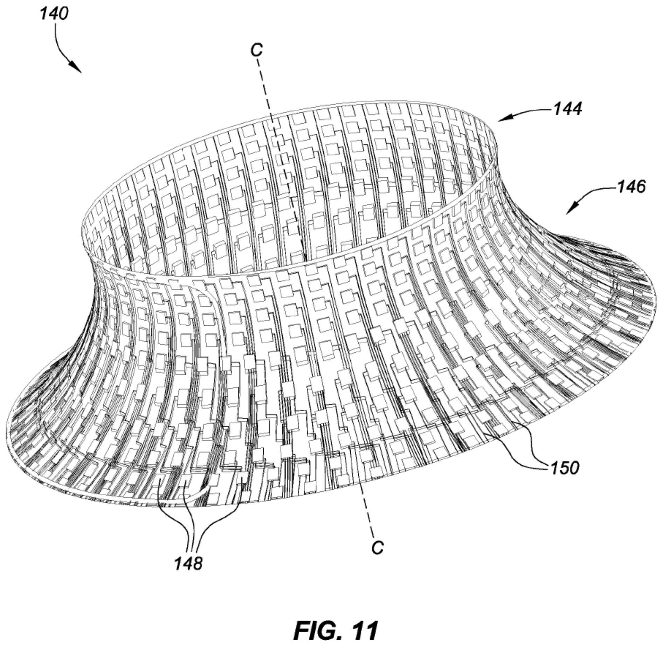

FIG. 11 is an isometric side and distal end view of a flexible catheter structure comprising an anatomically configured ablation tissue shaping element used to form the first shaping hoop and the second shaping hoop of FIG. 10, in accordance with embodiments of the present disclosure. The flexible substrate 146 is attached to the first shaping hoop 142 and second shaping hoop 144. Other support structures can be used instead of and/or in addition to a flexible substrate 146. The flexible substrate 146 can also be a braided material. The flexible substrate 146 can be a continuous piece of material and/or it can be a non-continuous piece of material (e.g., a web or lattice, or a combination of pieces). The flexible substrate 146 can include a plurality of interactive elements 148 for sensing tissue contact and/or force, temperature, electrical activity, position, and/or other desired characteristics.

The number of interactive elements on the flexible substrate 146 can vary. FIG. 11 shows the plurality of interactive elements 148 equally spaced covering the entire area between the first shaping hoop 142 and second shaping hoop 144. In some embodiments, the spacing between the plurality of interactive elements 148 can be varied. For example, the interactive elements nearest the first shaping hoop 142 (e.g., the larger diameter) can be closer to each other compared to the interactive elements nearest the second shaping hoop 144 (e.g., the smaller diameter). In some embodiments, the interactive elements can be arranged in lines or other configurations (e.g., one or more lines in a radial pattern, etc.) to facilitate treatment of tissue or data collection in specific locations relative to the placement of the first and second shaping hoops in the body.

An embodiment of FIG. 11, can have, for example, a first shaping hoop 142 with a diameter of 28.5 mm and a second shaping hoop 144 with a diameter of 18 mm. The distance between the first shaping hoop 142 and second shaping hoop 144 can be 7.5 mm (measured along an axis parallel to the line CC). In some embodiments, the interactive elements 148 can be 0.5mm long per side (e.g., a square configuration). The conductive electrical traces 150 can be, for example, 0.01 mm in thickness. Other suitable sizes for the interactive elements 148 can be used. The first and second shaping hoops 142 and 144 can be deployed or delivered using a deflectable catheter or a guidewire based delivery or other delivery method.

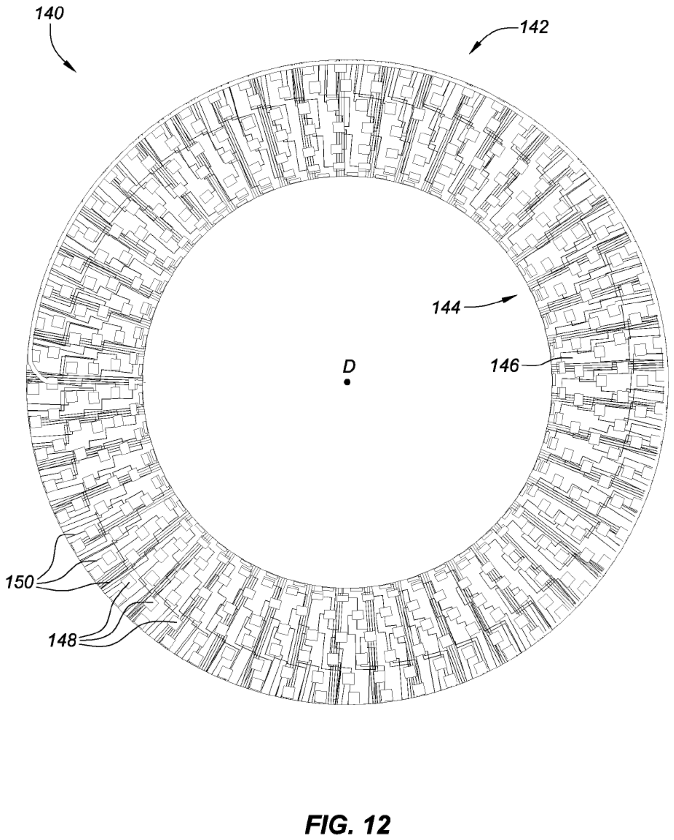

FIG. 12 is a proximal end view of the flexible catheter structure depicted in FIGS. 10 and 11, in accordance with embodiments of the present disclosure. An anatomically configured tissue shaping element 140 can be used to form the first shaping hoop 142 and the second shaping hoop 144, and the support structure 146 are centered on an axis defined by the line D (shown here as a single point as the axis is perpendicular to the surface of the paper with the drawing). The support structure 146 can be, for example, a flexible substrate, a braided material, a lattice, or a web.

The support structure 146 can also be shaped to extend between the first shaping hoop 142 and second shaping hoop 144 when they are aligned with an axis defined by the line D. For example, the support structure 146 can have a generally conical shape and/or flared conical shape with a curved surface (as described above) that can support a plurality of interactive elements 148 to facilitate contact between the plurality of interactive elements 148 and tissue (e.g., locations proximate the PV). The interactive elements 148 are further described herein. In some embodiments, the support structure 146 can also have a plurality of sensors that have a single function (e.g. measuring temperature, contact force, total force, strain, position, etc.). As described herein, contact between the interactive elements 148 and other sensors and tissue can allow for various treatment (e.g., ablation) or data gathering (e.g. measuring temperature, contact force, total force, strain, position, etc.).

The re-modeling of the PV when the first shaping hoop 142 and second shaping hoop 144 are in contact with the PV can cause the PV tissue to be in contact with a plurality of the interactive elements 148 located on the first shaping hoop 142 and second shaping hoop 144 (or on the support structure 146 with other sensors). The interactive elements 148 can allow for sensing and/or delivering energy to both the antrum and ostium/sleeve of the PV. The plurality of interactive elements 148 provide sufficient distribution to detect PV isolation. For example, the plurality of interactive elements 148 can be used to determine if sufficient ablation has been performed during a procedure to isolate the PV by sending test electrical signals into a first tissue location at a first interactive element and detecting electrical signals at a second tissue location.

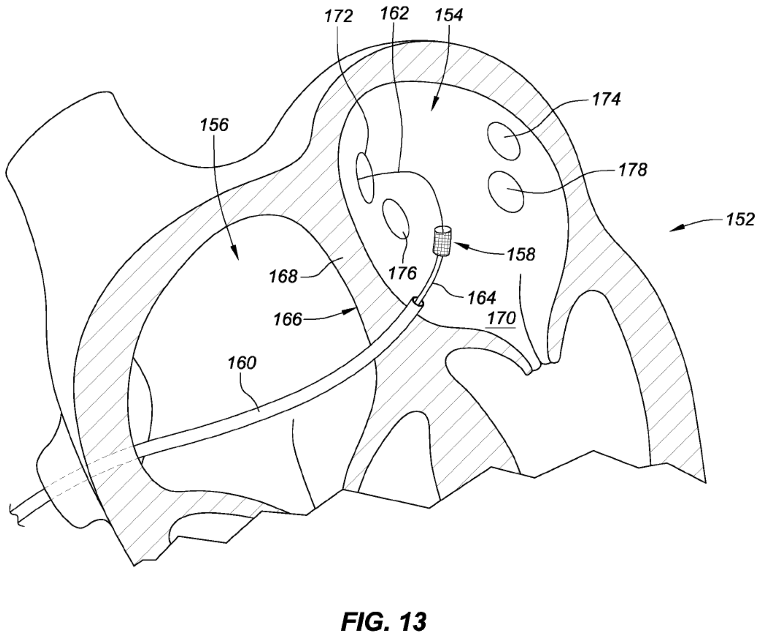

FIG. 13 is a cross-sectional front view of a portion of a heart with a catheter with the anatomically configured device of FIGS. 10-12 about to be located in a pulmonary vein, consistent with various aspects of the present disclosure. As shown in FIG. 13, the cardiac muscle 152 includes two upper chambers called the left atrium 154 and right atrium 156, and two lower chambers called the left ventricle and right ventricle (not shown).

As shown in FIG. 13, a tissue shaping device 158 may be introduced into the left atrium 154 by an introducer 160. A guidewire 162 and a catheter 164 may guide the tissue shaping device 158 once introduced into the left atrium 154 by the introducer 160. Optionally, the tissue shaping device 158 may include positioning sensors (e.g., mapping electrodes, not shown) at one or more locations of the tissue shaping device 158 as described herein. In operation, the introducer 160 can have its distal end positioned within the left atrium 154. As shown in FIG. 13, a transeptal approach may be utilized in which the introducer 160 is introduced through a peripheral vein (typically a femoral vein) and advanced to the right atrium 156. The introducer 160 can make a small incision into fossa ovalis 166 which allows the distal end of the introducer 160 to enter the left atrium 154 (through the transeptal wall 168) and to anchor itself to the wall of the fossa ovalis 166.

The tissue shaping device 158 may also be introduced into the left atrium 154 through the arterial system. In that case, the introducer 160 is introduced into an artery (such as a femoral artery) and advanced retrograde through the artery to the aorta, the aortic arch, and into the left ventricle. The tissue shaping device 158 can be extended from within a lumen of the introducer 160 to enter the left atrium 154 through mitral valve 170.

Once the introducer 160 is in position within the left atrium 154, the tissue shaping device 158 can be advanced out a distal end of the introducer 160 and toward one of the pulmonary veins (e.g., 172, 174, 176, and 178). In FIG. 13, the target pulmonary vein is right superior pulmonary vein 172. The guidewire 162 and a catheter 164 can be manipulated until the distal end of the tissue shaping device 158 can be directed toward the ostium of the target pulmonary vein, after the tissue shaping device 158 is extended into the pulmonary vein (e.g., superior pulmonary vein 172).

Carried near a distal end of the catheter 164, the tissue shaping device 158 can remain in a collapsed condition so that it may pass through introducer 160, and enter target pulmonary vein 172. Once in position, the tissue shaping device 158 can be deployed (e.g., expanded), so that it engages and secures the tissue shaping device 158 in a position axial to the target pulmonary vein 172 and in contact with tissue of the pulmonary vein 172.

The embodiment of FIG. 13 may include mapping electrodes (not shown). The mapping electrodes may be ring electrodes that allow the clinician to perform a pre-deployment electrical mapping of the conduction potentials of the pulmonary vein 172. Although the tissue shaping device 158 can include electrodes or sensors used for mapping, mapping electrodes or sensors may alternatively be carried on-board a separate electrophysiology catheter (not shown).

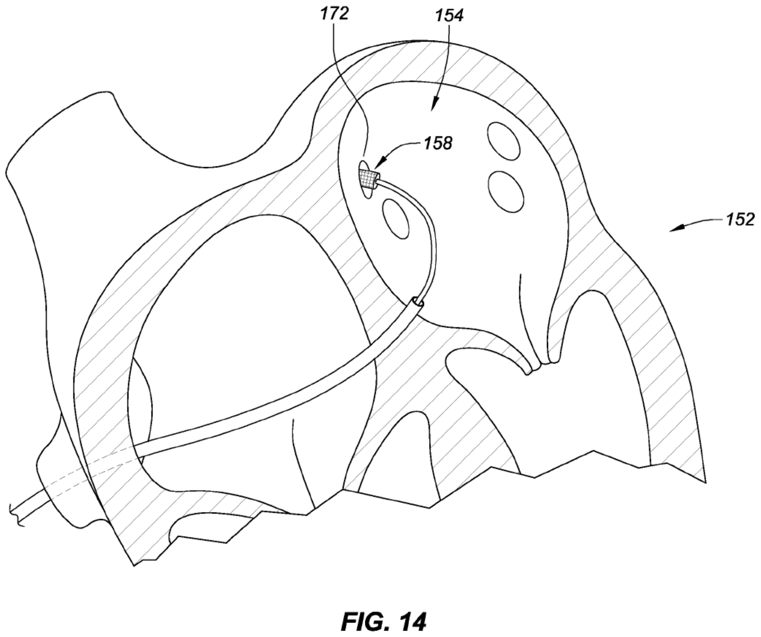

FIG. 14 is a cross-sectional front view of a heart with the with the flexible catheter structure comprising the anatomically configured tissue shaping element of FIGS. 10-12 positioned within the right superior pulmonary vein, prior to deployment of the tissue shaping element, in accordance with embodiments of the present disclosure.

FIG. 14 shows tissue shaping device 158 advanced into the ostium of pulmonary vein 172. As the tissue shaping device 158 enters the pulmonary vein 172, mapping may be conducted using position sensors/electrodes (not shown) in order to verify proper location prior to deployment of the tissue shaping device 158.

Aspects of the present disclosure can improve the fit of the tissue shaping device 158 within the pulmonary vein 172 with an anatomically configured device profile that betters conforms to the contours of the pulmonary vein 172 between antral and ostia portions thereof. This improved conformance between the expanded tissue shaping device 158 and pulmonary vein 172 can result in improved ablation therapy efficacy, and the reduced need for duplicative therapies.

In further example embodiments, the tissue shaping device 158 may be a specific to a particular pulmonary vein. For example, various studies have determined average, maximum, and minimum pulmonary vein diameters across various patient demographics. Using such data, anatomically configured devices for each of the pulmonary veins may be created and swapped out during a therapeutic procedure for atrial fibrillation patients, for example. Increasing efficacy of the ablation procedure. Various other parameters of a pulmonary vein may also be considered to tailor custom therapeutic solutions, thereby improving contact between each pulmonary vein and the tissue shaping device 158. In one specific example, where a range of diameters of a pulmonary vein ostia (e.g., right superior pulmonary vein) are between 15 and 20 millimeters, first portion of the tissue shaping device 158 may have a diameter around 19 millimeters to ensure contact (when inflated) between the pulmonary vein and the first portion for most patients, while limiting the potential for damage to smaller diameter pulmonary veins which may be permanently damaged by excess wall stress on the pulmonary vein tissue. Moreover, when the tissue is experiencing an excess wall stress, the ablation therapy can suffer from decreased efficacy and consistency of ablation.

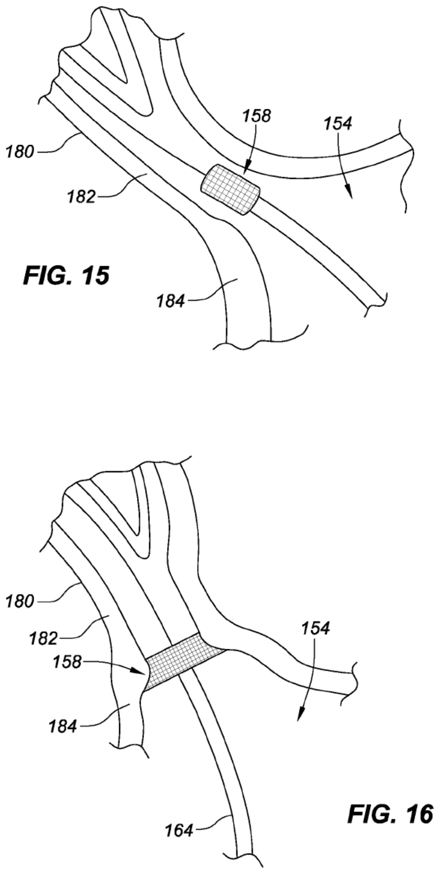

FIG. 15 is an enlarged cross-sectional front-view of a pulmonary vein with the anatomically configured device of FIGS. 10-12 positioned therein, prior to deployment of the anatomically configured device, in accordance with embodiments of the present disclosure. FIG. 15 shows tissue shaping device 158 in position within target pulmonary vein 180 prior to deployment of the tissue shaping device 158. In one embodiment of the present disclosure, the proper location of the , tissue shaping device 158 may be determined/verified by mapping, prior to deployment of the tissue shaping device 158. As shown in FIG. 15, ostial and antral portions of the pulmonary vein, 182 and 184 respectively, are irregular and varying in shape along both a longitudinal length and a cross-section of the pulmonary vein. Importantly, it has been discovered that many pulmonary veins exhibit an oval cross-sectional shape, as opposed to circular. Accordingly, where embodiments of the tissue shaping device 158 can be substantially circular, during expansion certain portions of the oval cross-sectional shape of the pulmonary vein may be overly stressed, while other portions of the pulmonary vein do not contact the anatomically configured device limiting, for example, efficacy of the ablation therapy. Accordingly, aspects of the present disclosure are directed to an anatomically configured device with a substantially oval shape. Such embodiments minimize and unify wall stress along a circumference of the pulmonary vein tissue.

FIG. 16 is a cross-sectional front view similar to that depicted in FIG. 15 but showing the anatomically configured tissue shaping element of FIGS. 10-12 deployed therein, in accordance with embodiments of the present disclosure. FIG. 16 shows expanded tissue shaping device 158 engaged between the ostial portion 182 and the antral portion 184 of the target pulmonary vein 180. In its expanded state shown in FIG. 13, tissue shaping device 158 engages inner walls of target pulmonary vein 180. The expanded shape of the tissue shaping device 158 can have distinct portions, designed to more precisely match the contours of the pulmonary vein. This distinct shape can increase the surface area contact between the pulmonary vein and the expanded tissue shaping device 158, which can improve, for example, the efficacy of an ablation therapy or other therapy that relies on surface contact between the tissue shaping device 158 and pulmonary vein tissue. Without continuous contact along a circumference of the pulmonary vein, a continuous lesion along the circumference may not be formed or, for other sensors, data may not be accurate or possible.

Once therapy (e.g., ablation, tissue cooling, etc.) is complete, the tissue shaping device 158 may be contracted and then retracted back into introducer 160. An electrophysiology catheter, or electrodes/sensors proximal and distal to the tissue shaping device 158, may be used to verify the efficacy of the therapy prior to removal of the tissue shaping device 158. In various embodiments of the present disclosure, and described herein, additional electrodes/sensors may also be positioned on the tissue shaping device 158, either alone, or in conjunction with the other sensors.

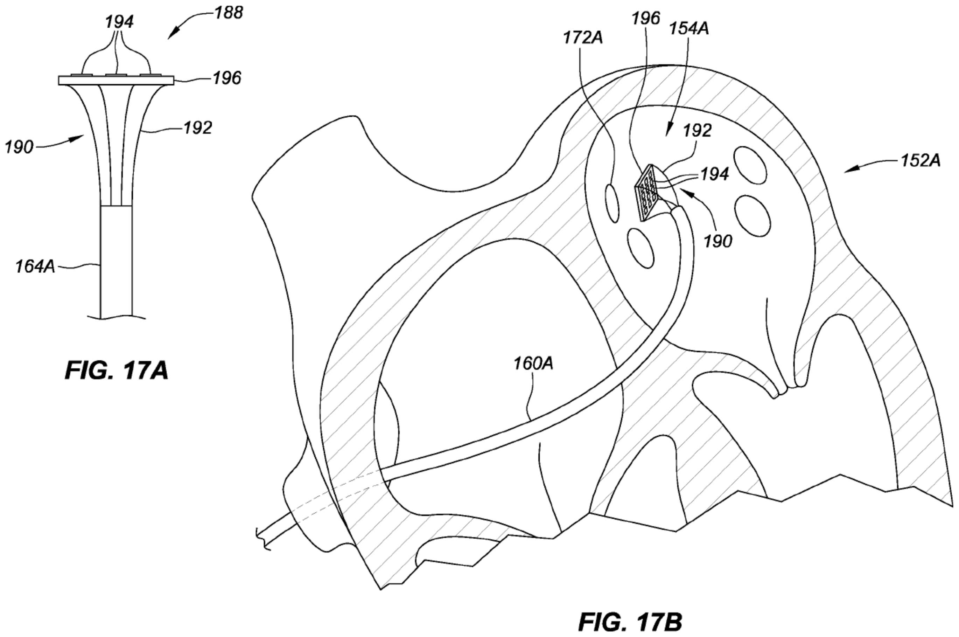

FIG. 17A is a side view of a flexible catheter structure comprising a planar array of sensors, in accordance with embodiments of the present disclosure. Tissue, (e.g., a heart 186) can have a need for a device that is shaped to fit different locations compared to openings of veins or similar structures in the heart 186. The planar array of sensors 188 can be used for diagnostic (e.g., mapping) or therapy (e.g., ablation) purposes in various locations of the heart 186. The planar array of sensors 188 can be formed from an expandable structure 190 (similar to descriptions above) that includes one or more struts 192 and a plurality of interactive elements 194 (described herein). The planar array of sensors 188 can include, for example, a support structure attached to the expandable structure 190 such as a flexible substrate 196.

In one embodiment, shown in FIG 17A, the expandable structure 190 can include four struts 192. Other embodiments can have a fewer or a greater number of struts 192. The struts 192 can be naturally biased to be in an expanded state (e.g., FIG. 17A). In another embodiment, the expandable structure 190 can be biased to be in a collapsed state (not shown). The expandable structure 190 can be made from self-erecting material (e.g., Nitinol) and/or other means can be used to expand the structure (e.g., pull wires, balloons, etc.). The struts 192 and the expandable structure 190 can be configured to orient the flexible structure 196 in a transverse orientation (e.g., perpendicular to the struts and generally perpendicular with a distal end of a catheter/introducer). In other embodiments (not shown), a planar array of sensors can have struts and an expandable structure configured to orient a flexible substrate to be parallel to a distal end of a catheter/introducer (e.g., co-extensive with the catheter/introducer).

The flexible substrate 196 can be formed from a continuous piece of material or it can be a non-continuous piece of material similar to a lattice or a web. The flexible substrate 196 can also be a braided material. The flexible substrate 196 can include a plurality of interactive elements 194 for sensing, for example, tissue contact and/or force, temperature, electrical activity, position, or other desired characteristics. The plurality of interactive elements 194 can be used for diagnostic (e.g., mapping) or therapy (e.g., ablation) purposes. The interactive elements 194 can be electrically connected (e.g., a plurality of conductive electrical traces, wires, etc.) to a power supply, controller, medical positioning system 14 or other device used to, for example, generate, amplify, receive, and/or process a signal. The transverse planar array of sensors 188 can be any suitable shape including, for example, square, circular, or rectangular. The transverse planar array of sensors 188 can be deployed using any suitable method. For example, the transverse planar array of sensors 188 can be rolled up into a cylindrical or tubular shape where the transverse planar array of sensors 188 can transform from a deployed state (shown in FIG. 17A) to a collapsed state (not shown) where the planar medical device is rolled to form a tube. The collapsed state can allow the planar medical device to fit inside, for example a shaft, a catheter, or an introducer. The shaft can be maneuvered to a desired location inside a body (e.g., the roof or wall of the heart). Once the shaft is at the desired location, the transverse planar array of sensors 188 can be deployed (e.g., buy pushing the transverse planar array of sensors 188 out of the shaft and/or by pulling the shaft proximally to expose the transverse planar array of sensors 188). When the transverse planar array of sensors 188 is positioned for use (e.g., deployed from the introducer 160A) the transverse planar array of sensors 188 can be generally perpendicular to a longitudinal axis of the introducer 160A.

The transverse planar array of sensors 188 can be manipulated so the flexible substrate 196 takes various shapes using any suitable method. For example, the planar medical device can be formed into a curved surface (e.g. convex or concave or some combination) using pull wires to adjust the shape of the expandable structure 190 and/or the flexible substrate 196. The variable shape of the transverse planar array of sensors 188 can allow the transverse planar array of sensors 188 to contact tissue at a variety of locations in a body (e.g., a heart). For example, the transverse planar array of sensors 188 can be used to provide therapy and/or treatment (e.g., ablation) to the roof, the walls, and/or the roof/wall interface of the heart. Similar to above, the flexible catheter structure 190 can be made from any suitable material, including Nitinol and other types of materials that have shape memory.

FIG. 17B is a cross-sectional side view of a graphical representation of a human heart with an embodiment of the transverse planar array of sensors depicted in FIG. 17A, in accordance with embodiments of the present disclosure. As described above, the transverse planar array of sensors 188 can include, for example, a support structure (e.g., struts 192) attached to the expandable structure 190 such as a flexible substrate 196. The transverse planar array of sensors 188 can be deployed and manipulated to be in contact with a portion of tissue, such as a portion of the heart 152A.

For example, the transverse planar array of sensors 188 can be deployed or delivered using a deflectable catheter (e.g., introducer 160A) or a guidewire (e.g., first catheter portion 162A) based delivery or other delivery method. The transverse planar array of sensors 188 can be positioned to facilitate contact between the interactive elements 194 (hidden from view in FIG. 17B) and/or the flexible substrate 196 and a portion of tissue (e.g., proximate the pulmonary vein 172A).

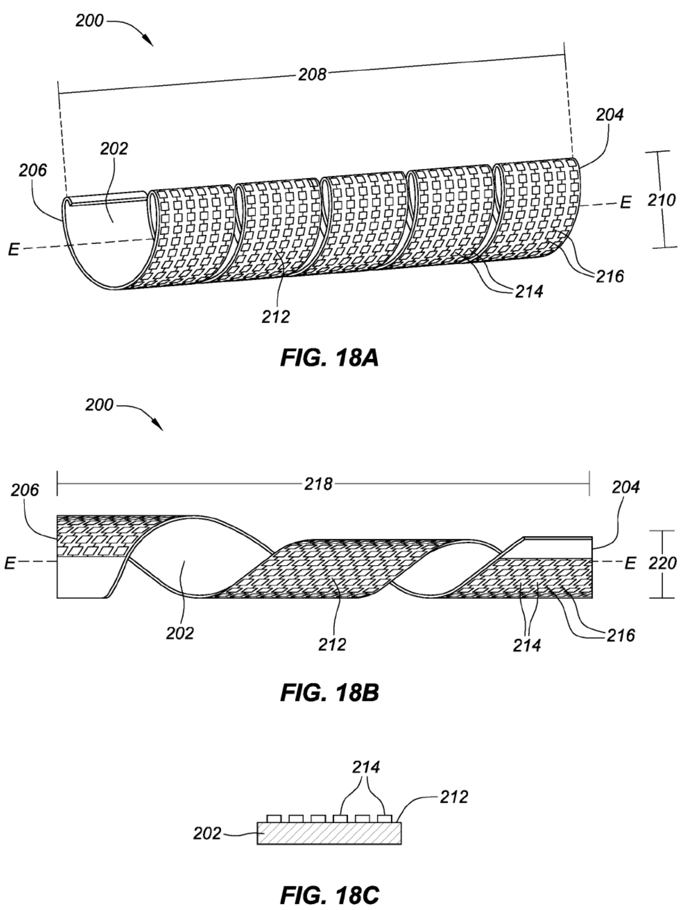

FIG. 18A is an isometric view of a helical medical device in an expanded state, in accordance with embodiments of the present disclosure. In some embodiments, the helical medical device 200 can include a first planar substrate 202 with a proximal end 204 and a distal end 206 and can extend along a longitudinal axis, defined by line EE. The first shape can be where a first length 208 of the helical medical device 200 is decreased between a proximal end 204 and a distal end 206 to increase an overall maximum diameter 210 or other dimension. An increased diameter (e.g., diameter 210) can facilitate contact between the helical medical device 200 and tissue. A decreased diameter can create a reduced delivery profile of the helical medical device 200 which can aid in placement over existing designs (see FIG. 18B). An increase in overall diameter can allow an increase in the outward force (e.g., perpendicular to the axis defined by the line EE) of the helical medical device 200 when in contact with tissue (e.g., the PV). The increase in outward force can create increased contact with the tissue surrounding the helical medical device 200.

The helical medical device 200 can be made from any suitable material (e.g., a polymer or metal) and can have any suitable material coating (e.g., a polymer coating over metal). For example, the helical medical device 200 can include the first planar substrate 202. The first planar substrate 202 can be a single continuous piece of material that is a rectangular strip formed into a helical structure as shown in FIG. 18A. The first planar substrate 202 can have an outer surface 212. The outer surface 212 can include a plurality of interactive elements 214 similar to above. The plurality of interactive elements 214 can be mounted to (e.g., coupled with) the first planar substrate 202 of the helical medical device 200 using any suitable method (e.g., adhesive, printed directly on the outer surface 212 using a printing process, deposited using an additive manufacturing process, etc.).

In some embodiments, a determination can be made whether or not the interactive elements are in contact with tissue based on an impedance signal generated by the interactive elements 214. It can be known if the interactive elements 214 are in contact with tissue or if the interactive elements 214 are not in contact with tissue (e.g., because of overlap of the helical medical device 200) by measuring, for example, impedance. If an interactive element 212 is on a first portion of the helical medical device 200 that is overlapped by a second portion of the helical medical device 200, an impedance measurement, for example, can be taken. If the impedance is, for example, zero or near zero (or some other value) the interactive elements 214 can be in contact with something other than tissue (e.g., the helical medical device 200 and/or other interactive elements 214).

The helical medical device 200 can include a plurality of conductive traces 216. The plurality of conductive traces 216 can be formed on the helical medical device 200 using any suitable printing technique (e.g., ink jet printing, additive manufacturing, etc.). The plurality of conductive traces 216 can connect, for example, the plurality of interactive elements 214. The electrical conductive traces 216 can be electrically connected (e.g., a plurality of conductive electrical traces, wires, etc.) to a power supply, controller, medical positioning system 14 or other device used to generate a signal. The helical medical device 200 can be used for diagnostic (e.g., mapping) or therapy (e.g., ablation) purposes.

The helical medical device 200 can be self-expanding. The helical medical device 200 can be designed so that it expands radially in a direction perpendicular to the axis defined by the line EE. A plurality of pull wires or other similar devices can be used to adjust the expansion of the helical medical device 200. For example, a pull wire at the distal end of the helical medical device 200 can be pulled in the proximal direction (e.g., longitudinally, or parallel to the line defined by the axis EE) towards the proximal end of the helical medical device 200 to add to the expansion force of the helical medical device 200 (e.g., force the distal end 206 closer to the proximal end 204, thus causing an increased expansion force to be applied to adjacent tissue). In another embodiment, a plurality of pull wires can be used to further adjust the shape of the helical medical device 200. For example a first pull wire can be used to adjust the expansion force at a first location (e.g., force the distal end 206 to be farther from to the proximal end 204, thus causing a decreased expansion force to be applied to adjacent tissue) on the helical medical device 200 and a second pull wire can be used to adjust the expansion force at a second location on the helical medical device 200. The use of multiple pull wires allows for a shape of the helical medical device to be tapered (e.g., different pull wires attached at different locations of the helical medical device 200 can allow a narrower diameter at the distal end and a wider diameter at the proximal end).

The tapered configuration of the helical medical device 200 can allow for better tissue contact (e.g., increased surface area of the helical medical device 200 in contact with the tissue and/or increased tissue contact forces) in certain locations. Other configurations are possible with additional pull wires (e.g., multiple tapered section). The expansion range of the helical medical device 200 can be, for example, 5-10 mm radially (perpendicular to the axis defined by the line EE). Other self-expanding geometries beyond the helical arrangement are possible. For example, self-expanding braded wire and non-self-expanding mechanisms such as trusses, struts, braid wire, or other similar structures.

FIG. 18B is an isometric view of the helical medical device of FIG. 18 in a contracted state, in accordance with embodiments of the present disclosure. The contracted state can be where the helical medical device 200 is stretched or lengthened from the length 208 to a second length 218 along an axis defined by the line EE to decrease (or contract) an overall diameter or other dimension and/or to decrease the outward force (e.g., force the distal end 206 to be farther from to the proximal end 204, thus causing a decreased expansion force to be applied to adjacent tissue). For example, the expansion force can be perpendicular to the axis defined by the line EE of the helical medical device 200. The decrease in outward force and a resulting diameter 220 (which can be less than maximum diameter 210) can aid in the movement and arrangement of the helical medical device 200 into an anatomical location of a body (e.g., the contracted state can allow the helical medical device to fit inside, for example a shaft, a catheter, or an introducer).

The helical medical device 200 can be placed using any suitable method. For example, the helical medical device 200 can be delivered to a tissue location using a guide wire, a catheter, an introducer, or a similar device. The guide wire diameter can be, for example, 5-8 French.

FIG. 18C is a cross-sectional view of a portion of the helical medical device of FIGS. 18A-B, in accordance with embodiments of the present disclosure. The interactive elements 214 can be coupled with the outer surface 212 of the first planar substrate 202.

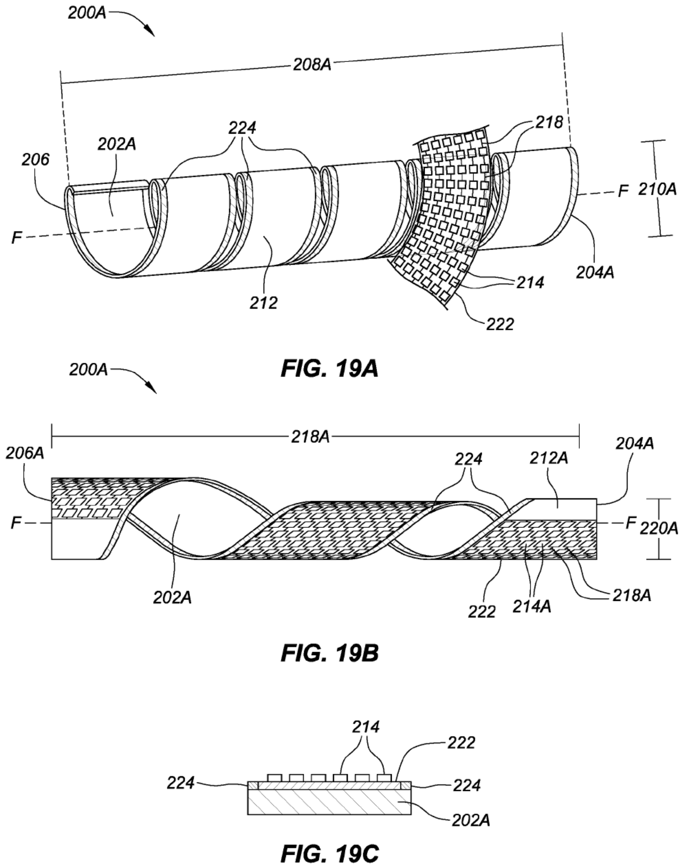

FIG. 19A is an isometric view of a helical medical device in an expanded state, in accordance with embodiments of the present disclosure. In this embodiment, the helical medical device 200A can include a flexible substrate 222 (e.g., a second planar substrate), edge material 224, and a plurality of interactive elements 214A. The plurality of interactive elements can be mounted to (e.g., coupled with) the flexible substrate 222 (e.g., a second planar substrate) where the flexible substrate 222 is attached to an outer surface 212A of a helical medical device 200A (e.g., a first planar substrate) using any suitable method. The flexible substrate 222 can be continuous along the outer surface 212A of the helical medical device 200A (e.g., one substrate that spirals along the entire length of the outer surface 212A of the helical medical device 200A from a proximal end 204A to a distal end 206A) or there may be a plurality of flexible substrates 222 along the outer surface 212A of the helical medical device 200A. In some embodiments, the flexible substrate 222 (e.g., the second planar substrate) can cover all or a portion of the outer surface 212A of the helical medical device 200A (e.g., the first planar substrate).

Similar to the embodiment described above in FIG. 18A, a contracted state can be where the helical medical device 200A is stretched or lengthened from the length 208A to a second length 218A along an axis defined by the line FF to decrease (or contract) an overall diameter or other dimension to decrease the outward force (e.g., force the distal end 206A to be farther from to the proximal end 204A, thus causing a decreased expansion force to be applied to adjacent tissue-see discussion related to FIG. 19B). For example, the expansion force can be perpendicular to the axis defined by the line FF of the helical medical device 200A. The decrease in outward force and the resulting diameter 220A (which can be less than maximum diameter 210A) can aid in the movement and arrangement of the helical medical device 200A into an anatomical location of a body (e.g., the contracted state can allow the helical medical device to fit inside, for example a shaft, a catheter, or an introducer).

As shown in FIG. 19A, the helical medical device 200A can also include an edge material 224. In some embodiments, the edge material 224 can be located proximate the edge of the helical medical device 200A as shown in FIG. 19A. The edge material 224 can be any suitable material, including nitinol and other types of materials that have shape memory. The edge material 224 can be any suitable shape including, for example, a flat or round wire. The edge material 224 can provide additional expansion forces in addition to expansion forces provided by the first planar substrate 202A and/or the second substrate 222. The additional expansion forces can assist with pressing the helical medical device 200A against tissue. The helical medical device 200A can be arranged to permit portions of the helical medical device 200A to overlap. The spiral/helical configuration can allow the helical medical device 200A to "spiral up" ((e.g., force the distal end 206A closer to the proximal end 204A, thus causing an increased expansion force to be applied to adjacent tissue) or expand to mate with various locations in a body (e.g., the outer diameter of a renal artery). Variations on the shape of the helical medical device can allow for better fit with body locations (e.g., tapered to a smaller diameter from a proximal to a distal end). Portions of the helical medical device 200A can overlap one another (e.g., when the helical medical device 200A is in an expanded state or a collapsed state).

The helical medical device 200A can be placed using any suitable method. For example, the helical medical device 200A can be delivered to a tissue location using a guide wire, a catheter, an introducer, or a similar device. The guide wire diameter can be, for example, 5-8 French.

In some embodiments, a determination can be made whether or not the interactive elements 214A are in contact with tissue based on an impedance signal generated by the interactive elements 214A. It can be known if the interactive elements 214A are in contact with tissue or if the interactive elements 214A are not in contact with tissue (e.g., because of overlap of the helical medical device 200A) by measuring, for example, impedance. If an interactive element 214A is on a first portion of the helical medical device 200A that is overlapped by a second portion of the helical medical device 200A, an impedance measurement, for example, can be taken. If the impedance is, for example, zero or near zero (or some other value) the interactive elements 214 can be in contact with something other than tissue (e.g., the helical medical device 200A, the flexible substrate 222 and/or other interactive elements 214A).

The helical medical device 200A can be self-expanding. The helical medical device 200A can be designed so that it expands radially in a direction perpendicular to the axis defined by the line FF. A plurality of pull wires or other similar devices can be used to adjust the expansion of the helical medical device 200A. For example, a pull wire at the distal end of the helical medical device 200A can be pulled in the proximal direction (e.g., longitudinally, or parallel to the line defined by the axis FF) towards the proximal end of the helical medical device 200A to add to the expansion force of the helical medical device 200A (e.g., force the distal end 206A closer to the proximal end 204A, thus causing an increased expansion force to be applied to adjacent tissue). In another embodiment, a plurality of pull wires can be used to further adjust the shape of the helical medical device 200. For example a first pull wire can be used to adjust the expansion force at a first location (e.g., force the distal end 206A to be farther from to the proximal end 204A, thus causing a decreased expansion force to be applied to adjacent tissue) on the helical medical device 200A and a second pull wire can be used to adjust the expansion force at a second location on the helical medical device 200A. The use of multiple pull wires allows for a shape of the helical medical device to be tapered (e.g., different pull wires attached at different locations of the helical medical device 200A can allow a narrower diameter at the distal end and a wider diameter at the proximal end).

The tapered configuration of the helical medical device 200A can allow for better tissue contact (e.g., increased surface area of the helical medical device 200A in contact with the tissue and/or increased tissue contact forces) in certain locations. Other configurations are possible with additional pull wires (e.g., multiple tapered section). The expansion range of the helical medical device 200A can be, for example, 5-10 mm radially (perpendicular to the axis defined by the line EE). Other self-expanding geometries beyond the helical arrangement are possible. For example, self-expanding braded wire and non-self-expanding mechanisms such as trusses, struts, braid wire, or other similar structures.

FIG. 19B is an isometric view of the helical medical device of FIG. 19A in a contracted state, in accordance with embodiments of the present disclosure. The contracted state can be where the helical medical device 200A is stretched or lengthened to a second length 218A along an axis defined by the line FF to decrease an overall diameter or other dimension and/or to decrease the outward force (e.g., force the distal end 206 to be farther from to the proximal end 204A, thus causing a decreased expansion force to be applied to adjacent tissue). For example, the expansion force can be perpendicular to the axis defined by the line FF of the helical medical device 200A. The decrease in outward force and a resulting diameter 220A (which can be less than maximum diameter 210A) can aid in the movement and arrangement of the helical medical device 200A into an anatomical location of a body (e.g., the contracted state can allow the helical medical device to fit inside, for example a shaft, a catheter, or an introducer).

FIG. 19C is a cross-sectional view of a portion of the helical medical device of FIGS. 19A-B, in accordance with embodiments of the present disclosure. The interactive elements 214A can be coupled with the second substrate 222 and the second substrate can be coupled with the first planar substrate 202A. The edge material 224 can be coupled with the first planar substrate 202A as shown in FIG. 19C. In other embodiments, the edge material 224 can be coupled with the second planar substrate 222 and then the combination can be coupled with the first substrate (not shown). The edge material 224 and the second substrate 222 can be any suitable size. In the embodiment shown in FIG. 19C, the edge material 224 and the second substrate 222 cover all of the outer surface 212A (at that particular portion) of the first planar substrate 202A. In other embodiments, portions of the outer surface 212A may not be covered by the edge material 224 and/or the second substrate 222 (e.g., there can be a gap between the edge material 224 and the second substrate 222).

Other structures or configurations are possible to facilitate locating and using the elements and structures described above. U.S. patent application nos. titled "Apparatuses and Methods for Cooling Tissue or Fluid," (attorney docket number CD-1133US/065513-001245) and "Apparatuses and Methods for Delivering Multiple Cardiac Ablations" (attorney docket number CD-1151US/065513-001512), both filed concurrently, are herein incorporated by reference in their entirety.

Various embodiments are described herein to various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," or "an embodiment", or the like, means that a particular feature, structure, or characteristic described in connection with the embodiment is included in at least one embodiment. Thus, appearances of the phrases "in various embodiments," "in some embodiments," "in one embodiment," or "in an embodiment", or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation given that such combination is not illogical or non-functional.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

The following is a list of numbered clauses of the invention:
1. A flexible catheter structure comprising:
   a first shaping element;
   a second shaping element located distally with respect to the first shaping element,
      wherein each of the first and the second shaping elements is expandable from a contracted state to an expanded state and are transversely oriented, when in the expanded state, with respect to a longitudinal axis that extends through a center of each shaping element;
   a support structure that extends between the first shaping element and the second shaping element; and
   an interactive element.
2. The flexible catheter structure of clause 1, wherein the interactive element is located on the support structure.
3. The flexible catheter structure of clause 1, wherein the interactive element is located on the first shaping element and a second interactive element is located on the second shaping element.
4. The flexible catheter structure of clause 1, further comprising a plurality of ring electrodes, wherein the plurality of ring electrodes are located on the first shaping element and the second shaping element.
5. The flexible catheter structure of clause 1, further comprising a flexible planar substrate, wherein the flexible planar substrate is connected to the first shaping element and the second shaping element and the interactive element is located on the flexible planar substrate.
6. The flexible catheter structure of clause 1, wherein the support structure is a braided material that extends between the first shaping element and the second shaping element.
7. The flexible catheter structure of clause 1, wherein the first shaping element and the second shaping element are formed from a single elongate element.
8. The flexible catheter structure of clause 1, wherein the first shaping element is formed from a first elongate element and the second shaping element is formed from a second elongate element.
9. The flexible catheter structure of clause 1, wherein the interactive element further comprises one or more of an energy delivery element, a thermocouple, a diagnostic element, a therapy element, a drug element, a chemical element, a biologic element, an acoustic element, an ultrasound element, a light-emitting element, a magnetic element, and a thermoelectric element.
10. The flexible catheter structure of clause 1, wherein the first and the second shaping elements each have a collapsed state and an expanded state, where the collapsed states are configured to fit inside a delivery device.
11. The flexible catheter structure of clause 1, wherein a width of the expanded state of the first shaping element is larger than a diameter of the delivery device and a width of the expanded state of the second shaping element is less than the width of the expanded state of the first shaping element.
12. The flexible catheter structure of clause 1, wherein one or more of the first shaping element, the second shaping element, and the support structure is formed from a shape memory material.
13. A flexible catheter structure comprising:
   a flexible substrate, wherein the flexible substrate is coupled with a distal end of the elongate medical device and is configured to have an expanded state and a collapsed state;
   a support structure, wherein the support structure is coupled with the flexible substrate; and
   a plurality of interactive elements, wherein the plurality of interactive elements are arranged in a pattern on the flexible substrate.
14. The apparatus of clause 13, wherein the pattern includes one of a plurality of interactive elements arranged in a plurality of longitudinal lines that extend from a distal end to a proximal end of the elongate medical device, a plurality of rows of interactive elements that are circumferentially disposed about an axis and longitudinally spaced apart of interactive elements centered about an axis, and a plurality of interactive elements arranged in a grid.
15. The apparatus of clause 13, wherein the plurality of interactive elements are electrically connected by a plurality of conductive traces, wherein the plurality of conductive traces are electrically connected to a power source.
16. The apparatus of clause 13, wherein the interactive elements further comprise one or more of an energy delivery element, a thermocouple, a diagnostic element, a therapy element, a drug element, a chemical element, a biologic element, an acoustic element, an ultrasound element, a light-emitting element, a magnetic element, and a thermoelectric element.
17. The apparatus of clause 13, further comprising an edge material, wherein the edge material is located proximal an edge of the flexible planar substrate and wherein the edge material is configured to expand the radius of the flexible planar substrate with respect to the longitudinal axis.
18. A helical medical device comprising:
   a flexible catheter structure comprising
   a first planar substrate, wherein the first planar substrate has a helical shape; and
   an interactive element, wherein the interactive element is coupled with the first planar substrate.
   a second planar substrate coupled with the first planar substrate, wherein the second planar substrate includes a plurality of interactive elements.
19. The helical medical device of clause 18, further comprising a second planar substrate, wherein the second planar substrate is between the first planar substrate and the interactive element.
20. The helical medical device of clause 19, wherein the second planar substrate covers a portion of the first substrate.
21. The helical medical device of clause 19, wherein the second planar substrate comprises a plurality of smaller planar substrates, wherein each of the plurality of smaller planar substrates include an interactive element.
22. The helical medical device of clause 20, wherein each of the interactive elements are electrically connected by a corresponding conductive trace, wherein the conductive traces are electrically connected to a power source.
23. The helical medical device of clause 19, wherein the interactive element is deposited onto at least one of the first planar substrate and the second planar substrate using a process selected from the group consisting of a printing process, an additive manufacturing process, and a deposition process.
24. An apparatus for an elongate medical device comprising:
   a support structure located at the shaft distal end, wherein the support structure is expandable from a contracted state to an expanded state with respect to the longitudinal axis;
   a flexible planar substrate coupled with the support structure at an outer surface formed by the support structure; and
   a plurality of interactive elements wherein the plurality of interactive elements are coupled with the flexible planar substrate.
25. The apparatus of clause 24, wherein the plurality of interactive elements further comprise an energy delivery element and one of a thermocouple, a force sensor, a strain gauge, a strain sensor, a position sensor, and a bio-sensor.
26. The apparatus of clause 24, wherein the support structure is formed from a braided material.
27. The apparatus of clause 24, wherein the support structure is formed from a shape memory material.
28. The apparatus of clause 24, wherein the support structure is configured to cause a tissue to conform to the expanded state of the support structure.
29. The apparatus of clause 24, wherein the expanded state of the support structure comprises a plurality of different diameters.
30. The apparatus of clause 24, wherein a number of the plurality of interactive elements is greater at a distal portion of the support structure compared to a proximal portion of the support structure.
31. The apparatus of clause 24, wherein the plurality of interactive elements are electrically connected by a plurality of conductive traces, wherein the plurality of conductive traces are electrically connected to a power source.
32. The apparatus of clause 24, wherein the plurality of interactive elements are deposited onto the support structure using a process selected from the group consisting of a printing process, an additive manufacturing process, and a deposition process.
33. The apparatus of clause 24, further comprising a plurality of ring electrodes, wherein the plurality of ring electrodes are located on the support structure.

## Claims

1. A flexible catheter structure comprising:
a first shaping element;
a second shaping element located distally with respect to the first shaping element,
wherein each of the first and the second shaping elements is expandable from a contracted state to an expanded state and are transversely oriented, when in the expanded state, with respect to a longitudinal axis that extends through a center of each shaping element;
a support structure that extends between the first shaping element and the second shaping element; and
an interactive element.

2. The flexible catheter structure of claim 1, wherein the interactive element is located on the support structure.

3. The flexible catheter structure of claim 1, wherein the interactive element is located on the first shaping element and a second interactive element is located on the second shaping element.

4. The flexible catheter structure of claim 1, further comprising a plurality of ring electrodes, wherein the plurality of ring electrodes are located on the first shaping element and the second shaping element.

5. The flexible catheter structure of claim 1, further comprising a flexible planar substrate, wherein the flexible planar substrate is connected to the first shaping element and the second shaping element and the interactive element is located on the flexible planar substrate.

6. The flexible catheter structure of claim 1, wherein the support structure is a braided material that extends between the first shaping element and the second shaping element.

7. The flexible catheter structure of claim 1, wherein the first shaping element and the second shaping element are formed from a single elongate element.

8. The flexible catheter structure of claim 1, wherein the first shaping element is formed from a first elongate element and the second shaping element is formed from a second elongate element.

9. The flexible catheter structure of claim 1, wherein the interactive element further comprises one or more of an energy delivery element, a thermocouple, a diagnostic element, a therapy element, a drug element, a chemical element, a biologic element, an acoustic element, an ultrasound element, a light-emitting element, a magnetic element, and a thermoelectric element.

10. The flexible catheter structure of claim 1, wherein the first and the second shaping elements each have a collapsed state and an expanded state, where the collapsed states are configured to fit inside a delivery device.

11. The flexible catheter structure of claim 1, wherein a width of the expanded state of the first shaping element is larger than a diameter of the delivery device and a width of the expanded state of the second shaping element is less than the width of the expanded state of the first shaping element.

12. The flexible catheter structure of claim 1, wherein one or more of the first shaping element, the second shaping element, and the support structure is formed from a shape memory material.
